# EUROPEAN PATENT APPLICATION

(11) **EP 2 767 548 A2**
(43) Date of publication of application: **20.08.2014**
(21) Application number: 14155839.5
(22) Date of filing: 19.02.2014
(51) Int. Cl.: C07K 16/00, C07K 16/28

(54) **Modified IgG molecules**

(30) Priority: 19.02.2013 GB 201302878
(71) Applicant: Argen-X B.V., 4811 AH Breda (NL)
(72) Inventor: De Haard, Johannes Joseph Wilhelmus, 4811 AH Breda (NL); Blanchetot, Christophe Frederic James, 4811 AH Breda (NL)
(74) Representative: Boult Wade Tennant

(57) **Abstract**

The present invention relates to modified IgG molecules with an Fc domain or a fragment thereof derived from IgG2, IgG3 or IgG4, and having specific combinations of altered and wild-type amino acid residues, in particular Lys433, Phe434 and a wild-type amino acid residue at position 436. The modified IgG molecules exhibit increased binding affinity for FcRn, as compared with wild-type IgG molecules of the corresponding subclass. The invention also relates to methods involving use of modified IgG molecules and pharmaceutical compositions and chimeric proteins comprising modified IgG molecules.

SEQUENCE LISTING

<110>  ARGEN-X B.V.

<120>  Modified IgG Molecules

<130>  NLW/JCP1/P126122EP00

<150>  GB1302878.2
<151>  2013-02-19

<160>  24

<170>  PatentIn version 3.5

<210>  1
<211>  50
<212>  DNA
<213>  Primer

<400>  1
gtgatgcatg aggctctgaa gttccactac acacagaaga gcctctccct          50


<210>  2
<211>  50
<212>  DNA
<213>  Primer

<400>  2
agggagaggc tcttctgtgt gtagtggaac ttcagagcct catgcatcac          50


<210>  3
<211>  50
<212>  DNA
<213>  Primer

<400>  3
gtgatgcatg aggctctgaa gttccgcttc acgcagaaga gcctctccct          50


<210>  4
<211>  50
<212>  DNA
<213>  Primer

<400>  4
agggagaggc tcttctgcgt gaagcggaac ttcagagcct catgcatcac          50


<210>  5
<211>  50
<212>  DNA
<213>  Primer

<400>  5
gtgatgcatg aggctctgaa gttccacttc acgcagaaga gcctctccct          50


<210>  6
<211>  50
<212>  DNA
<213>  Primer

<400>  6
agggagaggc tcttctgcgt gaagtggaac ttcagagcct catgcatcac                50

<210>  7
<211>  232
<212>  PRT
<213>  Homo sapiens

<400>  7

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  8
<211>  223
<212>  PRT
<213>  Homo sapiens

<400>  8

Val Glu Cys Pro Pro Cys Pro Ala Pro Pro Val Ala Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Met Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Gly Leu Pro Ala Pro Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220

<210>  9
<211>  242
<212>  PRT
<213>  Homo sapiens

<400>  9

Glu Ile Thr Gly Glu Phe Asp Ile Ser Ser Thr Met Val Arg Ser Asp
1               5                   10                  15

Thr Pro Pro Pro Cys Pro Arg Cys Pro Ala Pro Glu Leu Leu Gly Gly
            20                  25                  30

Pro Ser Val Phe Leu Phe Pro Pro Lys Pro Lys Asp Thr Leu Met Ile
        35                  40                  45

Ser Arg Thr Pro Glu Val Thr Cys Val Val Val Asp Val Ser His Glu
    50                  55                  60

Asp Pro Glu Val Gln Phe Lys Trp Tyr Val Asp Gly Val Glu Val His
65                  70                  75                  80

Asn Ala Lys Thr Lys Pro Arg Glu Glu Gln Tyr Asn Ser Thr Phe Arg
                85                  90                  95

Val Val Ser Val Leu Thr Val Leu His Gln Asp Trp Leu Asn Gly Lys
            100                 105                 110

Glu Tyr Lys Cys Lys Val Ser Asn Lys Ala Leu Pro Ala Pro Ile Glu
        115                 120                 125

Lys Thr Ile Ser Lys Thr Lys Gly Gln Pro Arg Glu Pro Gln Val Tyr
    130                 135                 140

Thr Leu Pro Pro Ser Arg Glu Glu Met Thr Lys Asn Gln Val Ser Leu
145                 150                 155                 160

Thr Cys Leu Val Lys Gly Phe Tyr Pro Ser Asp Ile Ala Val Glu Trp
                165                 170                 175

Glu Ser Ser Gly Gln Pro Glu Asn Asn Tyr Asn Thr Thr Pro Pro Met
            180                 185                 190

Leu Asp Ser Asp Gly Ser Phe Phe Leu Tyr Ser Lys Leu Thr Val Asp
        195                 200                 205

Lys Ser Arg Trp Gln Gln Gly Asn Ile Phe Ser Cys Ser Val Met His
    210                 215                 220

Glu Ala Leu His Asn Arg Phe Thr Gln Lys Ser Leu Ser Leu Ser Pro
225                 230                 235                 240

Gly Lys

<210>  10
<211>  224
<212>  PRT
<213>  Homo sapiens

<400>  10

Pro Pro Cys Pro Ser Cys Pro Ala Pro Glu Phe Leu Gly Gly Pro Ser
1               5                   10                  15

Val Phe Leu Phe Pro Pro Lys Pro Lys Asp Thr Leu Met Ile Ser Arg
            20                  25                  30

Thr Pro Glu Val Thr Cys Val Val Val Asp Val Ser Gln Glu Asp Pro
        35                  40                  45

Glu Val Gln Phe Asn Trp Tyr Val Asp Gly Val Glu Val His Asn Ala
    50                  55                  60

Lys Thr Lys Pro Arg Glu Glu Gln Phe Asn Ser Thr Tyr Arg Val Val
65                  70                  75                  80

Ser Val Leu Thr Val Leu His Gln Asp Trp Leu Asn Gly Lys Glu Tyr
                85                  90                  95

Lys Cys Lys Val Ser Asn Lys Gly Leu Pro Ser Ser Ile Glu Lys Thr
            100                 105                 110

Ile Ser Lys Ala Lys Gly Gln Pro Arg Glu Pro Gln Val Tyr Thr Leu
        115                 120                 125

Pro Pro Ser Gln Glu Glu Met Thr Lys Asn Gln Val Ser Leu Thr Cys
    130                 135                 140

Leu Val Lys Gly Phe Tyr Pro Ser Asp Ile Ala Val Glu Trp Glu Ser
145                 150                 155                 160

Asn Gly Gln Pro Glu Asn Asn Tyr Lys Thr Thr Pro Pro Val Leu Asp
                165                 170                 175

Ser Asp Gly Ser Phe Phe Leu Tyr Ser Arg Leu Thr Val Asp Lys Ser
            180                 185                 190

Arg Trp Gln Glu Gly Asn Val Phe Ser Cys Ser Val Met His Glu Ala
        195                 200                 205

Leu His Asn His Tyr Thr Gln Lys Ser Leu Ser Leu Ser Pro Gly Lys
    210                 215                 220

<210>  11
<211>  40
<212>  PRT
<213>  Homo sapiens

<400>  11

Val Leu Asp Ser Asp Gly Ser Phe Phe Leu Tyr Ser Lys Leu Thr Val
1               5                   10                  15

Asp Lys Ser Arg Trp Gln Gln Gly Asn Val Phe Ser Cys Ser Val Met
            20                  25                  30

His Glu Ala Leu His Asn His Tyr
        35                  40

<210>  12
<211>  40
<212>  PRT
<213>  Homo sapiens

<400>  12

Met Leu Asp Ser Asp Gly Ser Phe Phe Leu Tyr Ser Lys Leu Thr Val
1               5                   10                  15

Asp Lys Ser Arg Trp Gln Gln Gly Asn Val Phe Ser Cys Ser Val Met
            20                  25                  30

His Glu Ala Leu His Asn His Tyr
        35                  40

<210>  13
<211>  40
<212>  PRT
<213>  Homo sapiens

<400>  13

Met Leu Asp Ser Asp Gly Ser Phe Phe Leu Tyr Ser Lys Leu Thr Val
1               5                   10                  15

Asp Lys Ser Arg Trp Gln Gln Gly Asn Ile Phe Ser Cys Ser Val Met
            20                  25                  30

His Glu Ala Leu His Asn Arg Phe
        35                  40

<210>  14
<211>  40
<212>  PRT
<213>  Homo sapiens

<400>  14

Val Leu Asp Ser Asp Gly Ser Phe Phe Leu Tyr Ser Arg Leu Thr Val
1               5                   10                  15

Asp Lys Ser Arg Trp Gln Glu Gly Asn Val Phe Ser Cys Ser Val Met
            20                  25                  30

His Glu Ala Leu His Asn His Tyr
        35                  40

<210>  15
<211>  16
<212>  PRT
<213>  Homo sapiens

<400>  15

Val Met His Glu Ala Leu His Asn His Tyr Thr Gln Lys Ser Leu Ser
1               5                   10                  15

<210>  16
<211>  16
<212>  PRT
<213>  Mutant

<400>  16

Val Met His Glu Ala Leu Lys Phe His Tyr Thr Gln Lys Ser Leu Ser
1               5                   10                  15

<210>  17
<211>  20
<212>  PRT
<213>  Homo sapiens

<400>  17

Ser Cys Ser Val Met His Glu Ala Leu His Asn His Tyr Thr Gln Lys
1               5                   10                  15

Ser Leu Ser Leu
            20

<210>  18
<211>  20
<212>  PRT
<213>  Mutant

<400>  18

Ser Cys Ser Val Met His Glu Ala Leu Lys Phe His Tyr Thr Gln Lys
1               5                   10                  15

Ser Leu Ser Leu
            20



<210>  19
<211>  20
<212>  PRT
<213>  Homo sapiens

<400>  19

Ser Cys Ser Val Met His Glu Ala Leu His Asn Arg Phe Thr Gln Lys
1               5                   10                  15


Ser Leu Ser Leu
            20



<210>  20
<211>  20
<212>  PRT
<213>  Mutant

<400>  20

Ser Cys Ser Val Met His Glu Ala Leu Lys Phe Arg Phe Thr Gln Lys
1               5                   10                  15


Ser Leu Ser Leu
            20



<210>  21
<211>  20
<212>  PRT
<213>  Mutant

<400>  21

Ser Cys Ser Val Met His Glu Ala Leu Lys Phe His Phe Thr Gln Lys
1               5                   10                  15


Ser Leu Ser Leu
            20



<210>  22
<211>  20
<212>  PRT
<213>  Homo sapiens

<400>  22

Ser Cys Ser Val Met His Glu Ala Leu His Asn His Tyr Thr Gln Lys
1               5                   10                  15


Ser Leu Ser Leu
            20

<210>  23
<211>  20
<212>  PRT
<213>  Mutant

<400>  23

Ser Cys Ser Val Met His Glu Ala Leu Lys Phe His Tyr Thr Gln Lys
1               5                   10                  15

Ser Leu Ser Leu
            20

<210>  24
<211>  19
<212>  PRT
<213>  Homo sapiens

<400>  24

Met Gly Trp Ser Cys Ile Ile Leu Phe Leu Val Ala Thr Ala Thr Gly
1               5                   10                  15

Val His Ser

SEQUENCE LISTING

<110>  ARGEN-X B.V.

<120>  Modified IgG Molecules

<130>  NLW/JCP1/P126122EP00

<150>  GB1302878.2
<151>  2013-02-19

<160>  24

<170>  PatentIn version 3.5

<210>  1
<211>  50
<212>  DNA
<213>  Artificial Sequence

<220>
<223>  Primer

<400>  1
gtgatgcatg aggctctgaa gttccactac acacagaaga gcctctccct          50


<210>  2
<211>  50
<212>  DNA
<213>  Artificial Sequence

<220>
<223>  Primer

<400>  2
agggagaggc tcttctgtgt gtagtggaac ttcagagcct catgcatcac          50


<210>  3
<211>  50
<212>  DNA
<213>  Artificial Sequence

<220>
<223>  Primer

<400>  3
gtgatgcatg aggctctgaa gttccgcttc acgcagaaga gcctctccct          50


<210>  4
<211>  50
<212>  DNA
<213>  Artificial Sequence

<220>
<223>  Primer

<400>  4
agggagaggc tcttctgcgt gaagcggaac ttcagagcct catgcatcac          50

<210>  5
<211>  50
<212>  DNA
<213>  Artificial Sequence

<220>
<223>  Primer

<400>  5
gtgatgcatg aggctctgaa gttccacttc acgcagaaga gcctctccct                50


<210>  6
<211>  50
<212>  DNA
<213>  Artificial Sequence

<220>
<223>  Primer

<400>  6
agggagaggc tcttctgcgt gaagtggaac ttcagagcct catgcatcac                50


<210>  7
<211>  232
<212>  PRT
<213>  Homo sapiens

<400>  7

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  8
<211>  223
<212>  PRT
<213>  Homo sapiens

<400>  8

Val Glu Cys Pro Pro Cys Pro Ala Pro Pro Val Ala Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Met Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Gly Leu Pro Ala Pro Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220

<210>  9
<211>  242
<212>  PRT
<213>  Homo sapiens

<400>  9

Glu Ile Thr Gly Glu Phe Asp Ile Ser Ser Thr Met Val Arg Ser Asp
1               5                   10                  15

Thr Pro Pro Pro Cys Pro Arg Cys Pro Ala Pro Glu Leu Leu Gly Gly
            20                  25                  30

Pro Ser Val Phe Leu Phe Pro Pro Lys Pro Lys Asp Thr Leu Met Ile
        35                  40                  45

Ser Arg Thr Pro Glu Val Thr Cys Val Val Val Asp Val Ser His Glu
    50                  55                  60

Asp Pro Glu Val Gln Phe Lys Trp Tyr Val Asp Gly Val Glu Val His
65                  70                  75                  80

Asn Ala Lys Thr Lys Pro Arg Glu Glu Gln Tyr Asn Ser Thr Phe Arg
                85                  90                  95

Val Val Ser Val Leu Thr Val Leu His Gln Asp Trp Leu Asn Gly Lys
            100                 105                 110

Glu Tyr Lys Cys Lys Val Ser Asn Lys Ala Leu Pro Ala Pro Ile Glu
        115                 120                 125

Lys Thr Ile Ser Lys Thr Lys Gly Gln Pro Arg Glu Pro Gln Val Tyr
    130                 135                 140

Thr Leu Pro Pro Ser Arg Glu Glu Met Thr Lys Asn Gln Val Ser Leu
145                 150                 155                 160

Thr Cys Leu Val Lys Gly Phe Tyr Pro Ser Asp Ile Ala Val Glu Trp
                165                 170                 175

Glu Ser Ser Gly Gln Pro Glu Asn Asn Tyr Asn Thr Thr Pro Pro Met
            180                 185                 190

Leu Asp Ser Asp Gly Ser Phe Phe Leu Tyr Ser Lys Leu Thr Val Asp
        195                 200                 205

Lys Ser Arg Trp Gln Gln Gly Asn Ile Phe Ser Cys Ser Val Met His
    210                 215                 220

Glu Ala Leu His Asn Arg Phe Thr Gln Lys Ser Leu Ser Leu Ser Pro
225                 230                 235                 240

Gly Lys

<210>  10
<211>  224
<212>  PRT
<213>  Homo sapiens

<400>  10

Pro Pro Cys Pro Ser Cys Pro Ala Pro Glu Phe Leu Gly Gly Pro Ser
1               5                   10                  15

Val Phe Leu Phe Pro Pro Lys Pro Lys Asp Thr Leu Met Ile Ser Arg
            20                  25                  30

Thr Pro Glu Val Thr Cys Val Val Val Asp Val Ser Gln Glu Asp Pro
        35                  40                  45

Glu Val Gln Phe Asn Trp Tyr Val Asp Gly Val Glu Val His Asn Ala
    50                  55                  60

Lys Thr Lys Pro Arg Glu Glu Gln Phe Asn Ser Thr Tyr Arg Val Val
65                  70                  75                  80

Ser Val Leu Thr Val Leu His Gln Asp Trp Leu Asn Gly Lys Glu Tyr
                85                  90                  95

Lys Cys Lys Val Ser Asn Lys Gly Leu Pro Ser Ser Ile Glu Lys Thr
            100                 105                 110

Ile Ser Lys Ala Lys Gly Gln Pro Arg Glu Pro Gln Val Tyr Thr Leu
        115                 120                 125

Pro Pro Ser Gln Glu Glu Met Thr Lys Asn Gln Val Ser Leu Thr Cys
    130                 135                 140

Leu Val Lys Gly Phe Tyr Pro Ser Asp Ile Ala Val Glu Trp Glu Ser
145                 150                 155                 160

Asn Gly Gln Pro Glu Asn Asn Tyr Lys Thr Thr Pro Pro Val Leu Asp
                165                 170                 175

Ser Asp Gly Ser Phe Phe Leu Tyr Ser Arg Leu Thr Val Asp Lys Ser
            180                 185                 190

Arg Trp Gln Glu Gly Asn Val Phe Ser Cys Ser Val Met His Glu Ala
        195                 200                 205

Leu His Asn His Tyr Thr Gln Lys Ser Leu Ser Leu Ser Pro Gly Lys
    210                 215                 220

<210>  11
<211>  40
<212>  PRT
<213>  Homo sapiens

<400>  11

Val Leu Asp Ser Asp Gly Ser Phe Phe Leu Tyr Ser Lys Leu Thr Val
1               5                   10                  15

Asp Lys Ser Arg Trp Gln Gln Gly Asn Val Phe Ser Cys Ser Val Met
            20                  25                  30

His Glu Ala Leu His Asn His Tyr
        35                  40

<210>  12
<211>  40
<212>  PRT
<213>  Homo sapiens

<400>  12

Met Leu Asp Ser Asp Gly Ser Phe Phe Leu Tyr Ser Lys Leu Thr Val

1               5                   10                  15

Asp Lys Ser Arg Trp Gln Gln Gly Asn Val Phe Ser Cys Ser Val Met
            20                  25                  30

His Glu Ala Leu His Asn His Tyr
        35                  40

<210>  13
<211>  40
<212>  PRT
<213>  Homo sapiens

<400>  13

Met Leu Asp Ser Asp Gly Ser Phe Phe Leu Tyr Ser Lys Leu Thr Val
1               5                   10                  15

Asp Lys Ser Arg Trp Gln Gln Gly Asn Ile Phe Ser Cys Ser Val Met
            20                  25                  30

His Glu Ala Leu His Asn Arg Phe
        35                  40

<210>  14
<211>  40
<212>  PRT
<213>  Homo sapiens

<400>  14

Val Leu Asp Ser Asp Gly Ser Phe Phe Leu Tyr Ser Arg Leu Thr Val
1               5                   10                  15

Asp Lys Ser Arg Trp Gln Glu Gly Asn Val Phe Ser Cys Ser Val Met
            20                  25                  30

His Glu Ala Leu His Asn His Tyr
        35                  40

<210>  15
<211>  16
<212>  PRT
<213>  Homo sapiens

<400>  15

Val Met His Glu Ala Leu His Asn His Tyr Thr Gln Lys Ser Leu Ser
1               5                   10                  15

<210>  16
<211>  16
<212>  PRT

<213>  Artificial Sequence

<220>
<223>  Mutant

<400>  16

Val Met His Glu Ala Leu Lys Phe His Tyr Thr Gln Lys Ser Leu Ser
1               5                   10                  15


<210>  17
<211>  20
<212>  PRT
<213>  Homo sapiens

<400>  17

Ser Cys Ser Val Met His Glu Ala Leu His Asn His Tyr Thr Gln Lys
1               5                   10                  15


Ser Leu Ser Leu
            20


<210>  18
<211>  20
<212>  PRT
<213>  Artificial Sequence

<220>
<223>  Mutant

<400>  18

Ser Cys Ser Val Met His Glu Ala Leu Lys Phe His Tyr Thr Gln Lys
1               5                   10                  15


Ser Leu Ser Leu
            20


<210>  19
<211>  20
<212>  PRT
<213>  Homo sapiens

<400>  19

Ser Cys Ser Val Met His Glu Ala Leu His Asn Arg Phe Thr Gln Lys
1               5                   10                  15


Ser Leu Ser Leu
            20


<210>  20
<211>  20
<212>  PRT
<213>  Artificial Sequence

<220>
<223>  Mutant

<400>  20

Ser Cys Ser Val Met His Glu Ala Leu Lys Phe Arg Phe Thr Gln Lys
1               5                   10                  15

Ser Leu Ser Leu
            20

<210>  21
<211>  20
<212>  PRT
<213>  Artificial Sequence

<220>
<223>  Mutant

<400>  21

Ser Cys Ser Val Met His Glu Ala Leu Lys Phe His Phe Thr Gln Lys
1               5                   10                  15

Ser Leu Ser Leu
            20

<210>  22
<211>  20
<212>  PRT
<213>  Homo sapiens

<400>  22

Ser Cys Ser Val Met His Glu Ala Leu His Asn His Tyr Thr Gln Lys
1               5                   10                  15

Ser Leu Ser Leu
            20

<210>  23
<211>  20
<212>  PRT
<213>  Artificial Sequence

<220>
<223>  Mutant

<400>  23

Ser Cys Ser Val Met His Glu Ala Leu Lys Phe His Tyr Thr Gln Lys
1               5                   10                  15

Ser Leu Ser Leu
            20

<210>  24
<211>  19
<212>  PRT
<213>  Homo sapiens

<400>  24

Met Gly Trp Ser Cys Ile Ile Leu Phe Leu Val Ala Thr Ala Thr Gly
1               5                   10                  15

Val His Ser

## Description

### FIELD OF THE INVENTION

The present invention relates to immunoglobulin molecules and in particular, modified immunoglobulin G (IgG) molecules with amino acid substitutions conferring improved characteristics.

### BACKGROUND TO THE INVENTION

Immunoglobulin G (IgG) constitutes the most prevalent immunoglobin class in the serum of humans and other mammals. Several subclasses (or isotypes) of IgG exist in different species. In rat, IgG molecules belong to subclasses IgG1, IgG2a, IgG2b or IgG2c. The murine subclasses of IgG are IgG1, IgG2a, IgG2b and IgG3. Humans also have four subclasses of IgG: IgG1; IgG2; IgG3; and IgG4.

Despite fluctuations in rates of synthesis by B cells, IgGs are maintained at remarkably constant levels in the serum. If IgG homeostasis is disturbed, then pathology due to too high (hypergammaglobunemia) or too low (hypogammaglobunemia) can result. Studies indicate that the major histocompatibility complex (MHC)-class I related receptor, FcRn, is involved in the homeostasis of serum IgGs (Ghetie *et al,* 1996; Junghans and Anderson, 1996; Israel *et al,* 1996). This receptor most likely acts as a salvage receptor, and this would be consistent with its known ability to transcytose IgGs in intact form across the neonatal gut (Wallace and Rees, 1980; Rodewald and Kraehenbuhl, 1984) and yolk sac (Roberts *et al,* 1990; Israel *et al,* 1995) or placenta (Kristoffersen and Matre, 1996; Simister *et al,* 1996; Leach *et al,* 1996; Firan *et al,* 2001). More recent studies indicate that FcRn is also involved in the transport of IgGs across epithelial and endothelial cell barriers of diverse origin (Antohe *et al,* 2001; McCarthy *et al.,* 2000; Spiekermann *et al,* 2002; Dickinson *et al,* 1999; Kobayashi *et al,* 2002; Yoshida *et al,* 2004), and this has relevance to the delivery of IgG to different sites in the body. Thus, the use of protein engineering to modify the interaction site of an IgG with FcRn offers a way of modulating the serum persistence, distribution and transport of IgG molecules. The interaction sites of FcRn on mouse IgG1 (mIgG1) and human IgG1 (hIgG1) have been mapped using site-directed mutagenesis of recombinant Fc-hinge fragments, followed by analysis of these fragments both *in vivo* and *in vitro* (Kim *et al,* 1994b; Medesan *et al,* 1996; Medesan *et al,* 1997; Kim *et al,* 1999). From these studies, 1253 (EU numbering (Edehnan *et al,* 1969)), H310, H435 and to a lesser extent, H436 (Y436 in human IgG) play a central role in this interaction. These amino acids are typically conserved across IgG subclasses within species, and between IgG molecules found in rats, mice and humans (with the exception of H436/Y436). These residues are located at the CH2-CH3 domain interface (Deisenhofer, 1981), and the mapping of the functional site to these residues is consistent with the crystallographic structure of rat FcRn complexed with a rat Fc fragment (Burmeister *et al,* 1994b; Martin *et al,* 2001).

The FcRn interaction site within the Fc domain of IgG molecules encompasses three spatially close loops comprised of sequences that are distal in the primary amino acid sequence. The central role of Fc histidines in building this site accounts for the marked pH-dependence (binding at pH 6.0, release at pH 7.2-7.4) of the Fc-FcRn interaction (Rodewald and Kraehenbuhl, 1984; Raghavan *et al,* 1995; Popov *et al,* 1996), as the pKa of one of the imidazole protons lies in this pH range. This pH dependence is essential for the release of FcRn bound IgG molecules when they come to the cell surface following intracellular recycling or transcytosis (Ghetie and Ward, 2000; Ober *et al,* 2004a). As noted above, 1253, H310, H435 and to a lesser degree, H436, are highly conserved in IgGs of both human and rodent IgGs (Kabat *et al,* 1991). This, taken together with the isolation of a human homolog of FcRn (Story *et al,* 1994), indicate that the molecular mechanisms involved in IgG homeostasis and transport are common to both mouse and man and this has implications for the modulation of pharmacokinetics, distribution and delivery of IgGs to different body sites.

In studies to identify the FcRn interaction site on Fc, mutations of Fc fragments (comprising the Fc and hinge region) have been made that reduce the serum half-lives of the corresponding Fc fragments (Medesan *et al,* 1997; Kim *et al,* 1994a; Kim *et al,* 1999). In addition, Fc fragments or IgGs with increased affinity for binding to FcRn have been engineered (Ghetie *et al,* 1997; Shields *et al,* 2001; Hinton *et al,* 2004) and these molecules have increased serum persistence in mice (Ghetie *et al,* 1997) or cynomologous monkeys (Hinton *et al,* 2004). WO2006/130834 describes mutant IgG1 molecules having His433 changed to Lys and Asn434 changed to Phe, while Tyr436 is retained. These IgG1 molecules show a 6-fold higher binding affinity for human FcRn relative to wild-type, and retain pH-dependent binding to FcRn.

Immunoglobulin Fc domains are also of great interest for the purposes of studying the mechanisms of antibody interactions with further molecules of the immune system. These include, depending on the class of antibody, interactions with complement, and binding to specific receptors on other cells, including macrophages, neutrophils and mast cells. More detailed knowledge of the biology of Fc domains is important in understanding various molecular processes of the immune system, such as phagocytosis, antibody-dependent cell-mediated cytotoxicity (ADCC) and allergic reactions.

### SUMMARY OF THE INVENTION

The present invention relates to modified IgG molecules with an Fc domain or a fragment thereof derived from IgG2, IgG3 or IgG4, and having specific combinations of altered and wild-type amino acid residues. The modified IgG molecules exhibit increased binding affinity for FcRn, as compared with wild-type IgG molecules of the corresponding subclass.

In accordance with a first aspect of the invention, there is provided an IgG molecule comprising an Fc domain or a fragment thereof derived from IgG2, IgG3 or IgG4, wherein the Fc domain comprises Lys433, Phe434 and a wild-type amino acid residue at position 436. The Fc domain is preferably derived from IgG2, IgG3 or IgG4 of human origin. In embodiments wherein the Fc domain is derived from IgG2 or IgG4, the IgG molecule includes Tyr436. In embodiments wherein the Fc domain is derived from IgG3, the IgG molecule includes Phe436. The IgG molecule may further comprise His435. The numbering of all amino acid residues within the Fc domain is in accordance with the EU numbering scheme.

In certain embodiments, the Fc domain or fragment thereof may further comprise one or more of the following amino acid residues in any combination:- Tyr252, Thr254 and Glu256.

The modified IgG molecule according to the present invention may take any form provided the required Fc domain or fragment thereof is incorporated into the molecule. In certain embodiments, the IgG molecule of the present invention is selected from the following:- a bivalent immunoglobulin; a single chain immunoglobulin; a full-length IgG heavy chain polypeptide; and a fragment of an IgG heavy chain polypeptide. In embodiments wherein the IgG molecule is a fragment of an IgG heavy chain polypeptide, the fragment may be the full-length Fc domain of the IgG heavy chain polypeptide *i.e.* domains CH2 and CH3. Alternatively, the fragment may be at least 40, at least 50, at least 60, at least 70, at least 80, at least 90, at least 100, at least 110, at least 120, at least 130, at least 140, at least 150, at least 160, at least 170, at least 180, at least 190 or at least 200 amino acids in length. Any fragment should include at least amino acid residues 397 to 436 of the Fc domain of IgG according to EU numbering. The modified IgG molecules according to the present invention may be derived from human IgG2, IgG3 or IgG4 immunoglobulins.

In certain embodiments, the IgG molecule may include an Fv domain, or epitope-binding fragment thereof and therefore be capable of binding to an antigen, for example a pathogen antigen, or a toxin or other antibodies such as autoimmune or anti-transplant antibodies.

In certain embodiments, IgG molecules according to the present invention may be used as "delivery modules" through conjugation to further agents, for example therapeutic and/or diagnostic agents. The IgG molecules for conjugation are as defined above, and may in particular be a full-length Fc domain or fragment thereof. Suitable therapeutic agents may be selected from the following:- anti-bacterial; anti-viral and anti-toxin agents. Suitable diagnostic agents may be selected from a fluorescent label, a chemiluminescent label, a radiolabel, or a chromophore.

The IgG molecules as described herein may be formulated as a medicament for administration to a subject in need thereof. Furthermore, in accordance with a second aspect of the invention, there is provided a pharmaceutical composition comprising an IgG molecule as defined in the first aspect of the invention, and a pharmaceutically acceptable buffer, carrier, diluent or excipient. The IgG molecules or pharmaceutical compositions may be formulated for administration or delivery to a subject via an epithelium that expresses FcRn. The epithelium may be selected from the bronchial, intestinal or pulmonary epithelium.

In further aspects of the invention, provided herein are various methods of therapy utilising the IgG molecules according to the first aspect of the invention or the pharmaceutical compositions according to the second aspect of the invention. These methods of therapy include a method of treating an autoimmune disease or disorder, a method of treating infection with a pathogenic agent, and a method of treating cancer. Such methods involve administering to a subject in need thereof an effective amount of an IgG molecule or a pharmaceutical composition comprising the same according to the present invention.

Further provided herein are methods of increasing the serum half life of an IgG molecule, methods of increasing IgG clearance rates in a subject, methods of blocking FcRn function in a subject and methods of providing IgG molecules to a fetus. All the methods described use IgG molecules according to the first aspect of the invention.

In a further aspect of the invention, there is provided a chimeric protein comprising an IgG molecule as defined in the first aspect of the invention.

It is contemplated that any method or composition described herein can be implemented with respect to any other method or composition described herein. The use of the word "a" or "an" when used in conjunction with the term "comprising" in the claims and/or the specification may mean "one," but it is also consistent with the meaning of "one or more," "at least one," and "one or more than one." "About" means plus or minus 5% of the stated value.

These, and other, embodiments of the invention will be better appreciated and understood when considered in conjunction with the following description and the accompanying drawings. It should be understood, however, that the following description, while indicating various embodiments of the invention and numerous specific details thereof, is given by way of illustration and not of limitation. Many substitutions, modifications, additions and/or rearrangements may be made within the scope of the invention without departing from the spirit thereof, and the invention includes all such substitutions, modifications, additions and/or rearrangements.

### BRIEF DESCRIPTION OF THE DRAWINGS

**Figure 1** Immunoglobulin structure showing heavy and light chain polypeptides and the variable (V_{L} and V_{H}) and constant domains (CH1, CH2 and CH3) of each.
**Figure 2** **A.** Alignment of the Fc domain sequences from human IgG1, IgG2, IgG3 and IgG4. The numbering of amino acid sequences uses the EU numbering system.
The shaded regions represent FcRn binding regions (according to Strohl 2009, Current opinion on Biotechnology 20:685-691). The amino acid residues shown in bold represent differences between the four IgG subtypes. There is an allelic mutation in IgG2 at position 378. **B.** Alignment of Fc domain fragments from human IgG1, IgG2,
IgG3 and IgG4 spanning amino acid residues 397-436.

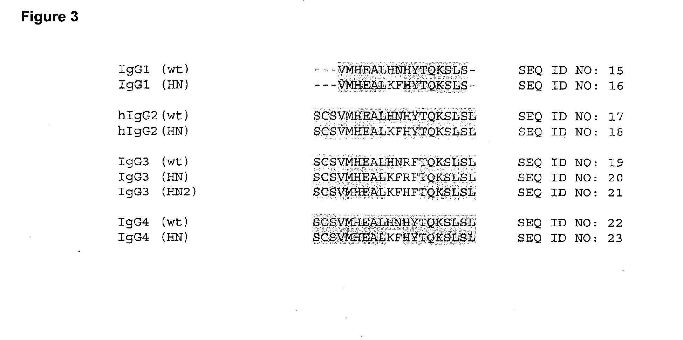

**Figure 3** Amino acid sequence of the different IgG variants (wild-type) and the mutations introduced in the different IgG subclasses.

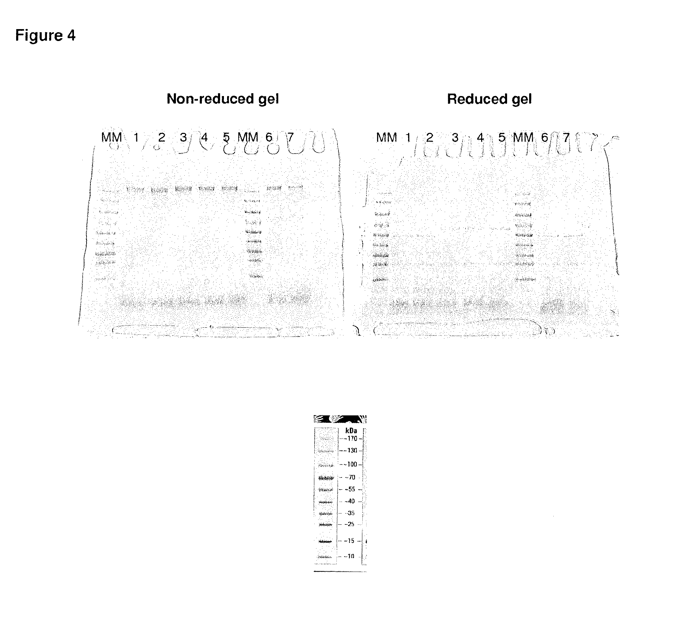

**Figure 4** SDS-PAGE analysis of the different MAB1 mAbs under non-reduced (left panel) and reduced (right panel) conditions. Lane 1: IgG2-MAB1; lane 2: IgG2-MAB1-HN; lane 3: IgG3-MAB1; lane 4: IgG3-MAB1-HN; lane 5: IgG3-MAB1-HN2; lane 6: IgG4-MAB1; lane 7: IgG4-MAB1-HN.

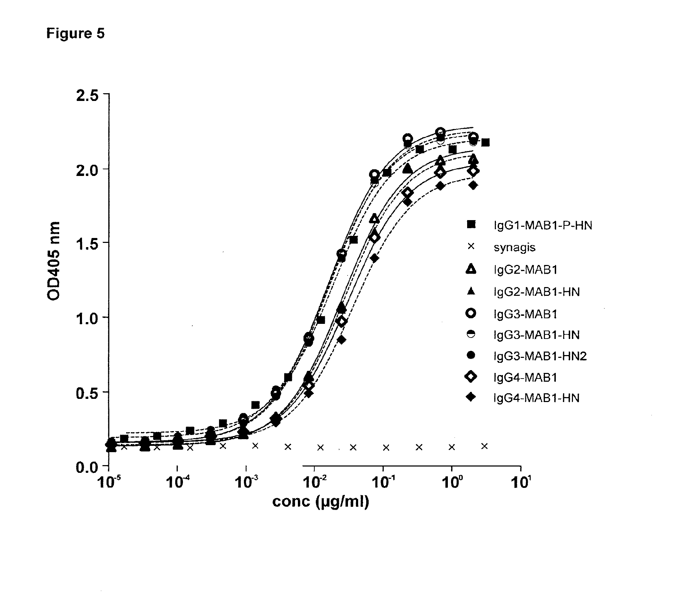

**Figure 5** Human Decoy Met binding ELISA for the different mAbs. The WT mAbs are represented by solid lines and the HN(2) mutated mAbs are represented by dashed lines.

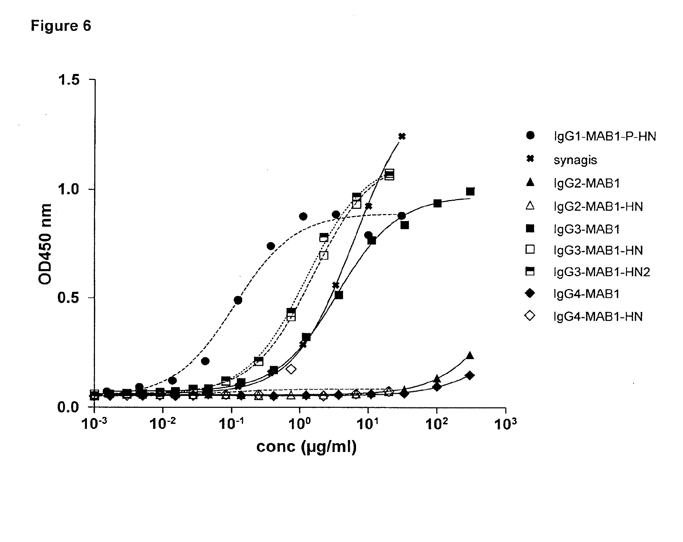

**Figure 6** Binding of the different mAbs to human CD16 in ELISA. The WT mAbs are represented by solid lines and the HN(2) mutated mAbs are represented by dashed lines.

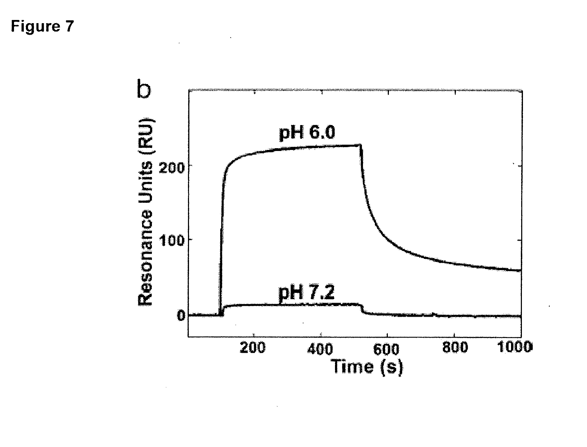

**Figure 7** Sensogram of FcRn interaction with HN mutant at pH6 and pH7. (Vaccaro C et al, PNAS, 103, 2006)

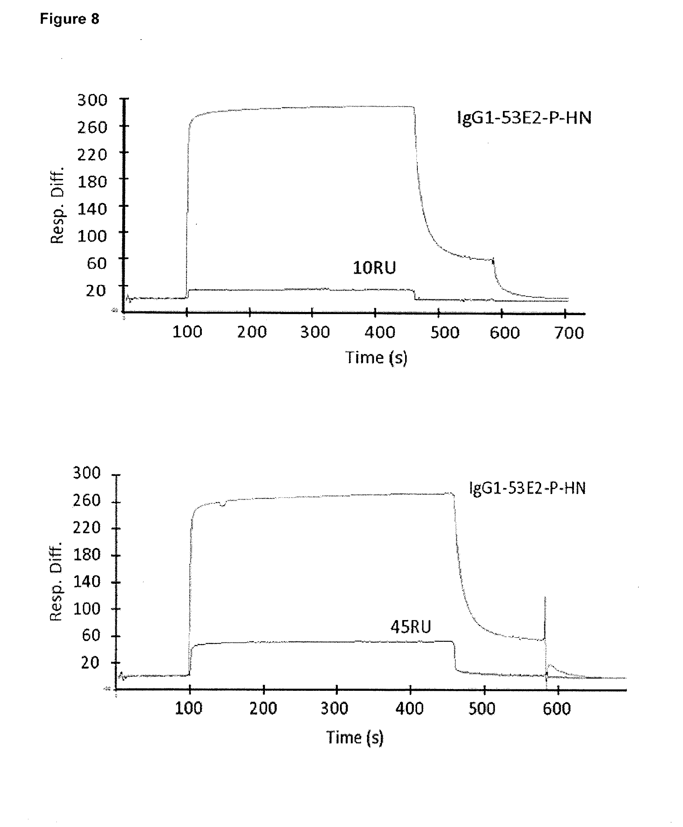

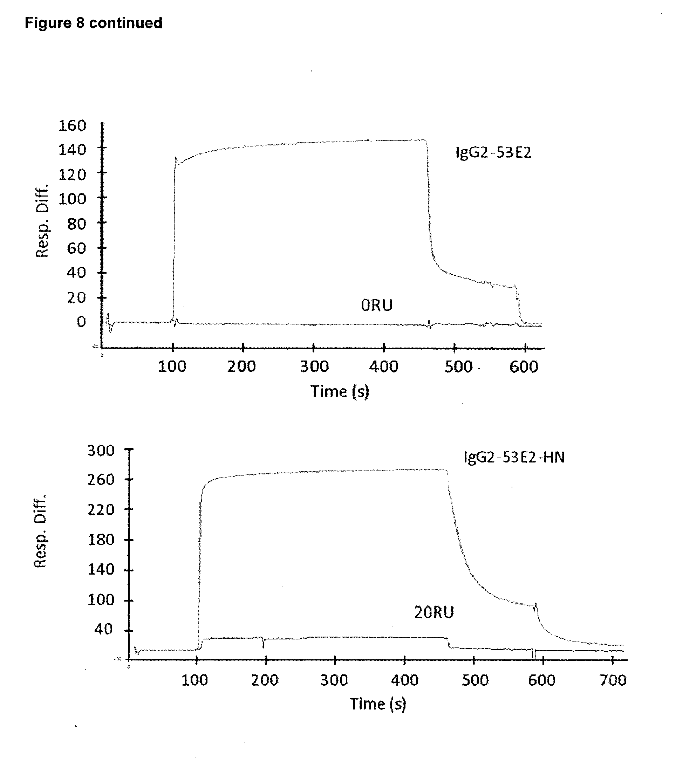

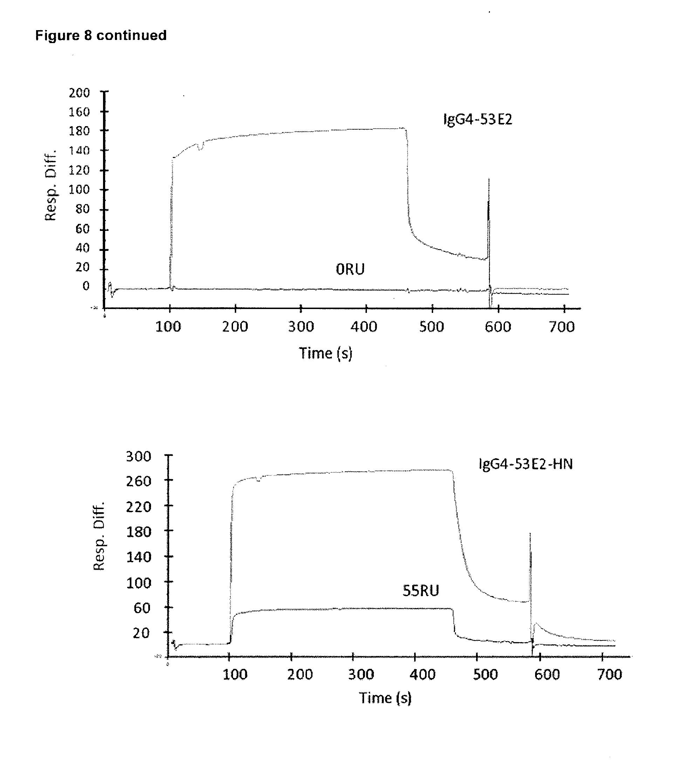

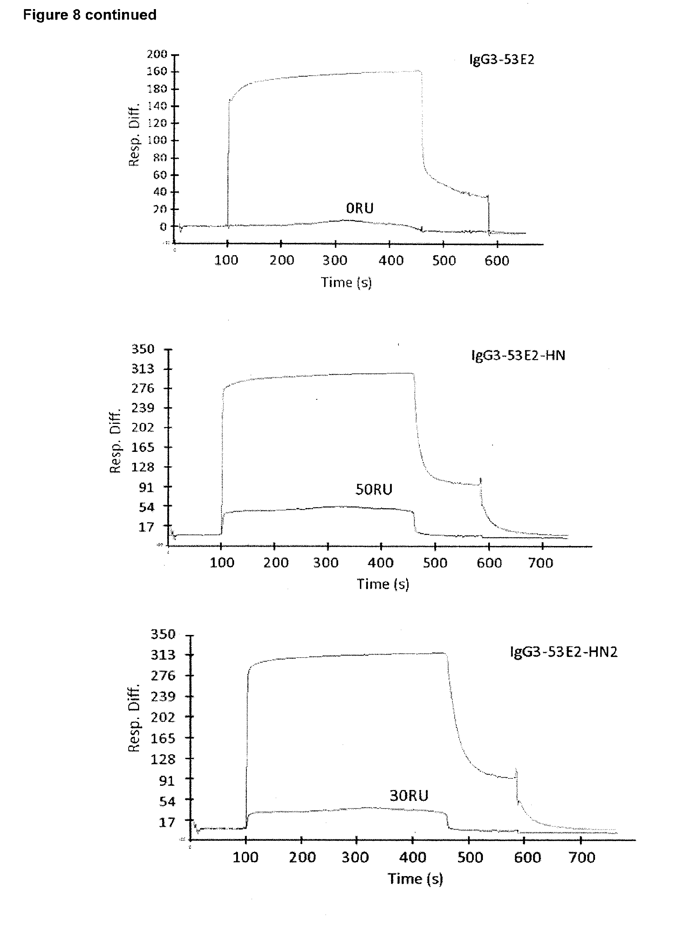

**Figure 8** Sensograms of 4µM FcRn interaction with MAB1 variants at pH6 (upper line) and pH7 (lower line).

### DETAILED DESCRIPTION

In a first aspect, the present invention concerns IgG molecules modified so as to have improved characteristics. Improved characteristics include but are not limited to improved binding to the FcRn receptor, increased serum half life, improved distribution to certain tissues including skin, muscle and brain and/or improved transplacental transfer or transport across other cellular barriers, as compared with wild-type or native IgG molecules.

The invention also concerns the generation of modified IgG molecules that have the ability to block FcRn function by virtue of enhanced binding to FcRn. More specifically, the invention is exemplified by the production of various mutant IgG molecules having a beneficial combination of altered and wild-type residues within the Fc domain.

The IgG molecules of the invention comprise an Fc domain or a fragment thereof derived from an IgG molecule belonging to IgG subclasses 2, 3 or 4. The IgG molecule from which the Fc domain derives may be of murine or rat origin, but is preferably derived from IgG2, IgG3 or IgG4 of human origin. The Fc domain of the IgG molecules of the invention comprises the following amino acid residues: Lys433; Phe434; and a wild-type amino acid residue at position 436 based on the EU numbering scheme for IgG molecules. As used herein, the term "wild-type amino acid residue" means the amino acid located at position 436 in the naturally-occurring IgG molecule from which the Fc domain or fragment thereof derives. In naturally-occurring human IgG2 and IgG4 molecules, position 436 is a Tyrosine, and in naturally-occurring human IgG3 molecules, position 436 is a Phenylalanine.

### 1. Antibody Constant Domains

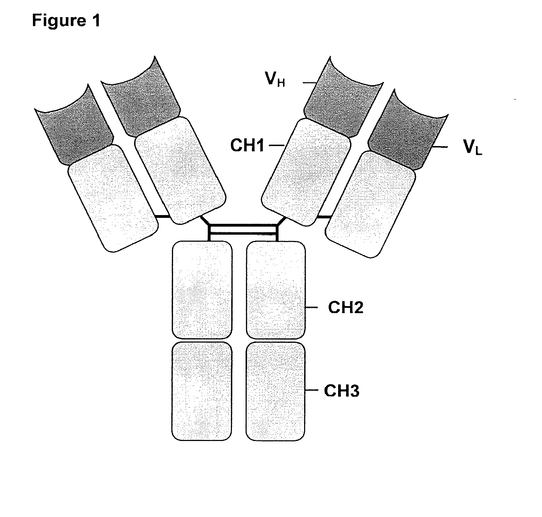

Native antibody or immunoglobulin molecules typically have a structure as depicted in Figure 1 with two identical heavy polypeptide chains of molecular weight 53,000-70,000 Da and two identical light polypeptide chains of molecular weight approximately 23,000 Da. The four chains are linked by disulphide bonds so as to form a "Y" configuration. Each heavy chain and each light chain has a variable domain (V_{H} and V_{L}), and hypervariable regions within these domains can combine to create two sites of antigen recognition. The heavy chains and light chains also include constant domains, and the heavy chain has three constant domains (C_{H}) identified as CH1, CH2 and CH3.

The Fc domain of an immunoglobulin consists of the second and third C_{H} domains (CH2-CH3) and is typically recognised by effector molecules of the immune system and cells carrying Fc receptors.

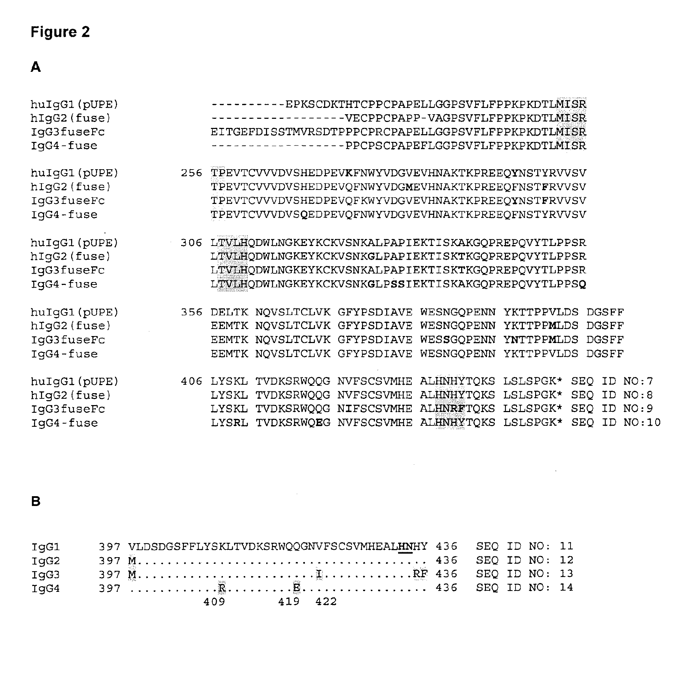

In certain embodiments, the IgG molecules of the present invention comprise an Fc domain derived from IgG2, IgG3 or IgG4, preferably wherein the IgG2, IgG3 or IgG4 is of human origin. Alternatively, IgG molecules of the present invention may comprise a fragment of an Fc domain derived from IgG2, IgG3 or IgG4, wherein a fragment is defined as a region of the Fc domain including at least residues 433-436 and comprising at least 40, at least 50, at least 60, at least 70, at least 80, at least 90, at least 100, at least 110, at least 120, at least 130, at least 140, at least 150, at least 160, at least 170, at least 180, at least 190 or at least 200 amino acids taken from the Fc domain of the originating IgG molecule. In embodiments wherein the IgG molecule comprises a fragment of an Fc domain, the minimum region of the Fc domain included in the fragment should be from amino acid residues 397 to 436 according to EU numbering (see Figure 2B).

The term "IgG molecule" insofar as it is used to describe modified IgG molecules according to the present invention is to be construed broadly so as to include a bivalent immunoglobulin; a single chain immunoglobulin; a full-length IgG heavy chain polypeptide; and a fragment of an IgG heavy chain polypeptide.

As used herein, a fragment of an IgG heavy chain polypeptide means a fragment having a length of at least 40, at least 50, at least 60, at least 70, at least 80, at least 90, at least 100, at least 110, at least 120, at least 130, at least 140, at least 150, at least 160, at least 170, at least 180, at least 190 or at least 200 amino acids. In certain embodiments, a fragment of an IgG heavy chain polypeptide may comprise a full-length Fc domain *i.e.* the CH2 and CH3 domains.

### 2. Regulation of IgG levels by FcRn

The maintenance of serum IgG concentrations at a fairly constant level is of importance for effective immunity. Moreover, abnormally high (hypergammaglobulinemia) or low (hypogammaglobulinemia) serum IgG levels result in clinical symptoms. To be effective, the homeostatic mechanism that both senses and regulates serum IgG levels must be able to deal with continuous and variable production of IgG molecules by the B cells of the organism. How such homeostasis is brought about is as yet unclear, and several mechanisms have been proposed to account for the control of IgG levels in the serum (Brambell *et al.,* 1964; Brambell, 1966; Ghetie *et al.,* 1981). Clearly, any model must invoke a feedback system that is both sensitive and responsive to changes in serum IgG levels. In addition, the transport of IgGs across cellular barriers is an important process in mediating protective humoral immunity, and is most likely closely related to the processes that regulate serum IgG levels.

The features of an immunoglobulin molecule that determine stability and other properties *in vivo* have been the subject of much study. The current model for how IgG levels are regulated in the body is that the Fc receptor, FcRn, binds to IgGs and transports them within and across cells.

The receptor FcRn has been isolated from duodenal epithelial brush borders of suckling rats (Rodewald and Kraehenbuhl, 1984; Simister and Rees, 1985) and the corresponding genes cloned for both human and mouse FcRn (Simister and Mostov, 1989a; Simister and Mostov, 1989b; Ahouse *et al.,* 1993). This Fc receptor comprises a heterodimer of two polypeptides of 51 kDa and 14 kDa. Interestingly, the 14 kDa component is β2-microglobulin and the 51 kDa component is homologous to the heavy chain of Class I MHC proteins. The protein can be expressed in high yields in recombinant form and has been analyzed by x-ray crystallography (Burmeister *et al.,* 1994a; Burmeister *et al.,* 1994b; Martin *et al,* 2001). Importantly, FcRn orthologs have been isolated from multiple species including humans (e.g., Story *et al.,* 1994; Kacskovics *et al.,* 2000), indicating that FcRn is present across mammalian species.

Binding to FcRn by naturally occurring IgG is pH dependent, with much tighter binding at pH 6.0 than at pH 7.2-7.4 (Raghavan *et al.,* 1995; Popov *et al,* 1996; Zhou *et al.,* 2003). In most cell types the pH at the cell surface is near neutral and as a result, non-permissive for FcRn-IgG interactions. IgGs are therefore taken into cells by fluid phase pinocytosis rather than receptor-mediated uptake. If, following uptake by fluid phase pinocytosis, IgGs do not bind to FcRn then they undergo lysosomal degradation (Ober *et al.,* 2004b). Following recycling or transcytosis by FcRn, IgGs are released by exocytosis at the cell surface (Ober *et al.,* 2004a). Thus, and consistent with the earlier model proposed by Brambell and colleagues prior to the identification of FcRn and the elucidation of the molecular mechanisms of FcRn function (Brambell *et al,* 1964; Brambell, 1966), the cells that are responsible for IgG breakdown are paradoxically also involved in protection of IgGs against breakdown. FcRn is expressed at multiple sites throughout the body (e.g., endothelium, epithelium, monocytes, dendritic cells), and therefore serves not only to regulate IgG levels by acting as a salvage receptor (reviewed in Ghetie and Ward, 2000), but also plays a role in transporting IgG across cellular barriers (Antohe *et al,* 2001; McCarthy *et al,* 2000; Spiekerman *et al,* 2002; Dickinson *et al,* 1999; Kobayashi *et al,* 2002; Yoshida *et al,* 2004). Consistent with the idea that FcRn plays a central role in regulating serum IgG levels, IgGs or Fc fragments are cleared rapidly from the circulation of mice that are deficient in functional FcRn expression (Ghetie *et al.,* 1996; Junghans and Anderson, 1996; Israel *et al,* 1996). Conversely, increasing the affinity of an IgG for FcRn would be predicted to increase its serum half life, and IgG mutants with improved serum half-life have been described (U.S. Patent 6,277,375; Ghetie *et al,* 1996, International patent application no. WO2006/130834).

### 3. FcRn Interaction Site on the IgG Molecule

Of the five Ig classes found in humans (IgA, IgE, IgM, IgD and IgG), IgG has the longest *in vivo* half life (Zuckier *et al,* 1990). The region of the IgG molecule that regulates serum persistence has been known for several decades to reside in the Fc fragment. This work, carried out initially by Spiegelberg and Weigle (1966) and later confirmed by many others (reviewed in Zuckier *et al,* 1990), indicated that the Fc fragment produced by proteolysis has the same *in vivo* half life as the complete IgG molecule. Works by Dorrington and colleagues (Dorrington and Painter, 1974; Ellerson *et al,* 1976; Yasmeen *et al,* 1976) showed that a CH2 domain fragment produced by trypsin digestion had the same half life as that of the complete IgG molecule. Although both earlier and more recent data suggest that the CH2 domain is involved in the regulation of serum persistence of IgGs, some of these data are not inconsistent with the additional involvement of the CH3 domain (Arend and Webster, 1977; Dima *et al,* 1983; Mueller *et al,* 1990; Kim *et al,* 1994a; Batra *et al,* 1993). Indeed, other work has indicated that the CH2 domain, and to a lesser extent the CH3 domain, contain sequences that control the serum persistence of IgG molecules (Kim *et al,* 1994a; Pollock *et al,* 1990, Kim *et al,* 1994c). In particular, site-directed mutagenesis has been used to identify amino acid residues in the CH2-CH3 domain interface that are critical for the maintenance of serum IgGI levels in mice (Kim *et al,* 1994a; Medesan *et al,* 1997), and these studies therefore resulted in the precise localization of the FcRn binding site.

The residues involved in binding to FcRn are highly conserved in both human and murine IgG isotypes (Kim *et al,* 1994a; Table I), and analyses of human IgG molecules (or Fc fragments) and mutated variants indicate that similar IgG residues are involved in binding to FcRn in humans (Kim *et al,* 1999; Shields *et al,* 2001).

**Table 1**

| | **252-254** | **308-312** | **433-436³** |
|---|---|---|---|
| mIgG1¹ | TIT | IMHQD | HNHH |
| mIgG2a | MIS | IQHQD | HNHH |
| mIgG2b | MIS | IQHQD | KNYY |
| mIgG3 | MIS | IQHQD | HNHH |
| hIgG1² | MIS | VLHQD | HNHY |
| hIgG2 | MIS | VVHQD | HNHY |
| hIgG3 | MIS | VLHQD | HNRF |
| hIgG4 | MIS | VLHQD | HNHY |

| | | | |
|---|---|---|---|
| ¹mIgG1 = murine IgG1; ²hIgG1 = human IgG1; ³Simultaneous mutation of His433 and Asn434 had an effect, but as single mutations, His433 to Ala433 and Asn434 to Ala434, no effect was observed (Medesan *et al.* 1997). Residues of murine IgG1 or human IgG1 that were mutated and found to affect clearance rates in mice (Kim *et al.,* 1994a; Kim *et al.,* 1999) are underlined. | | | |

The mapping of the FcRn interaction site on IgG by site directed mutagenesis is also consistent with the high resolution X-ray crystallographic structure of rat FcRn complexed with rat Fc (IgG2a), which demonstrated that residues 252, 253, 254, 288, 307, 309, 310,311, 314, 348, 433, 434, 435, 436 and 439 of IgG make contact to varying extents with FcRn (including carbohydrate contacts of FcRn on Fc) (Martin *et al,* 2001).

The involvement of His310, His435 and His436 (Tyr in human IgG) of the IgG molecule in interacting with FcRn explains, in part at least, the pH dependence of the FcRn-Fc interaction (Kim *et al,* 1994b; Raghavan *et al,* 1995). Despite similarities of the FcRn interaction sites on IgGs across species, recent studies have shown that human and mouse FcRn can show differences in binding specificity (Ober *et al,* 2001). Site directed mutagenesis has recently been used to determine the molecular basis for this (Zhou *et al,* 2003; 2005). The region of the Fc that is involved in binding to FcRn (Kim *et al.,* 1994a; Medesan *et al.,* 1997; Martin *et al.,* 2001) is distinct from the sites involved in binding FcγRI, RII and RIII receptors (the "classical" FcRs), as these recognize sequences primarily located in the lower hinge region (Duncan *et al,* 1988; Lund *et al,* 1992; Sarmay *et al,* 1992; Jefferis *et al,* 1990; Canfield and Morrison, 1991; Wawrzynczak *et al,* 1992). In addition, the FcRn interaction site is distinct from the complement factor Clq binding site (Glu318, Lys320 and Lys322) (Wawrzynczak *et al,* 1992; Duncan and Winter, 1988). Thus, mutation of the FcRn interaction site should neither affect complement fixation nor binding to FcγRI, RII and RIII.

### 4. Sequence Differences at the Interaction Site on the CH3 Domain of IgG

Of the IgG residues shown in Table I that are involved in binding to FcRn, the most marked differences are in the CH3 domain. This allows sequence differences to be correlated with possible functional differences. For example, murine IgG2b has been shown to have a more rapid clearance rate than IgG1, IgG2a and IgG3 (Pollock *et al,* 1990). Analysis of sequence differences for the residues at the CH2-CH3 domain interface that have been shown to be important in building the FcRn interaction site indicate that in murine IgG2b, His433, His435, His436 of murine IgG1, IgG2a and IgG3 are replaced by Lys433, Tyr435 and Tyr436 (Table I).

These sequence differences provide an ideal system to analyze the role of position 433, 435 and 436 in controlling serum half life and IgG transport. The conversion of lysine 433 to histidine 433, tyrosine 435 to histidine 435 and tyrosine 436 to histidine 436 in an IgG2b molecule results in a mutated IgG2b that has the same *in vivo* half-life as murine IgGl (U.S. Patent 6,277,375). However, comparative binding studies of mouse IgGI and mouse IgG2b indicate that these sequence differences result in a loss of pH dependence of the corresponding FcRn-IgG interaction, rather than a reduced affinity (Raghavan *et al,* 1995; Zhou *et al,* 2003). This, together with studies of Dall'Acqua *et al* (2002), indicate that reduced pH dependence can result in decreased serum persistence, most likely due to inefficient release of IgG molecules from cell associated FcRn during exocytosis (Ober *et al,* 2004a).

Individual mutation of His433 to Ala and Asn434 to Ala/Gln has no effect on binding to FcRn, serum half life or transcytosis (U.S. Patent 6,277,375; Medesan et al, 1997), although His433 and Asn434 have been shown to contact carbohydrate on FcRn in the rat FcRn-Fc complex structure (Martin *et al,* 2001). In earlier studies (Kim *et al,* 1994a; 1994c), it was noted that double mutation of His433, Asn434 of mouse IgG1 did have a moderate effect on FcRn-mediated functions in mice. This variation is most likely due to the perturbation of adjacent critical residues such as His435 by the double mutation (whereas single mutations are less perturbing) rather than central involvement of 433 and 434 in the FcRn-IgG (or Fc) interaction. These residues are therefore in proximity to residues that are essential for FcRn-IgG interactions, and are good potential targets for affinity improvement (using a similar strategy to that described in Ghetie *et al,* 1997) when IgG residues flanking the key residue 253 were targeted for mutagenesis.

### 5. Modified IgG molecules with improved FcRn binding properties

The present invention is concerned with IgG molecules comprising an Fc domain or fragment thereof derived from IgG2, IgG3 or IgG4 wherein the Fc domain comprises the the particular combination of amino acid residues Lys433, Phe434 and a wild-type amino acid residue at position 436 (mutant/mutant/wild-type).

As used herein, the term "derived" refers to the origin of the sequence of the Fc domain and means that the Fc domain or fragment thereof has a sequence of amino acids corresponding to the sequence of amino acids found in the corresponding Fc domain of an IgG subclass selected from IgG2, IgG3 or IgG4. In certain embodiments, the IgG molecule from which the Fc domain or fragment thereof derives is of human origin. Wherein the IgG molecule is human IgG2 or IgG4, the wild-type amino acid residue at position 436 is tyrosine. Wherein the IgG molecule is human IgG3, the wild-type amino acid residue at position 436 is phenylalanine. Optionally, amino acid residue 435 may be further mutated to histidine wherein the IgG molecule is human IgG3.

This combination provides for at least a 3-fold, typically a 4-6 fold increase in the affinity of IgG molecule binding to human FcRn at pH6.0 as compared with binding of the corresponding wild-type IgG to human FcRn at pH6.0. Binding of IgG molecules to FcRn at pH7.4 is largely unaffected therefore the pH dependence of the interaction between IgG and FcRn is retained for the modified IgG molecules according to the present invention. In addition, the affinity of binding to CD16 is not reduced for IgG molecules or antibodies incorporating the combination of mutations according to the present invention and therefore effector function is retained. This is important for the triggering of effector mechanisms such as antibody-dependent cell-mediated cytotoxicity (ADCC) by the modified IgG molecules described herein.

In certain embodiments, the modified IgG molecules of the invention may include one or more additional amino acid residues selected from:- Tyr252, Thr254 and Glu256.

### 6. Polynucleotides, DNA constructs, and Host Cells

The invention also provides polynucleotide molecules encoding the modified IgG molecules of the invention, expression vectors containing these nucleotide sequences operably linked to regulatory sequences which permit expression of the IgG molecules in a host cell or cell-free expression system, and host cells or cell-free expression systems containing these expression vectors.

Polynucleotide molecules encoding the modified IgGs of the invention include, for example, recombinant DNA molecules. The terms "nucleic acid", "polynucleotide" or a "polynucleotide molecule" are used herein interchangeably and refer to any DNA or RNA molecule, either single- or double-stranded and, if single-stranded, the molecule of its complementary sequence. In discussing nucleic acid molecules, a sequence or structure of a particular nucleic acid molecule may be described herein according to the normal convention of providing the sequence in the 5' to 3' direction. In some embodiments of the invention, nucleic acids or polynucleotides are "isolated." This term, when applied to a nucleic acid molecule, refers to a nucleic acid molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or non-human host organism.

An isolated polynucleotide (either DNA or RNA) may further represent a molecule produced directly by biological or synthetic means and separated from other components present during its production. For recombinant production of an IgG molecule according to the invention, recombinant polynucleotide encoding the IgG molecule may be prepared (using standard molecular biology techniques) and inserted into a replicable vector for expression in a chosen host cell, or a cell-free expression system. Suitable host cells may be prokaryote, yeast, or higher eukaryote cells, specifically mammalian cells. Expression vectors suitable for use in each of these host cells are also generally known in the art.

It should be noted that the term "host cell" generally refers to a cultured cell line (prokaryote or eukaryote). Whole human beings into which an expression vector encoding an IgG molecule according to the invention has been introduced are explicitly excluded from the definition of a "host cell".

### 7. Mutagenesis

In accordance with the present invention the mutagenesis of polynucleotides encoding IgG Fc regions can be performed using site-specific or site-directed mutagenesis. Site-specific mutagenesis is a technique useful in the preparation of individual proteins or peptides, or biologically functional equivalent proteins or peptides, through specific mutagenesis of the underlying DNA. The technique further provides a ready ability to prepare and test sequence variants, incorporating one or more of the foregoing mutations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to 25 nucleotides in length is preferred, with about 5 to 10 residues on both sides of the junction of the sequence being altered.

In general, the technique of site-specific mutagenesis is well known in the art. As will be appreciated, the technique can employ a bacteriophage vector that exists in both a single stranded and double stranded form. Typical vectors useful in site-directed mutagenesis include vectors such as the M13 phage. These phage vectors are commercially available and their use is generally well known to those skilled in the art. Double stranded plasmids are also routinely employed in site directed mutagenesis, using the PCR™, which eliminates the step of transferring the gene of interest from a phage to a plasmid.

In general, site-directed mutagenesis is performed by first obtaining a single-stranded vector, or melting of two strands of a double stranded vector which includes within its sequence a DNA sequence encoding the desired protein. An oligonucleotide primer bearing the desired mutated sequence is synthetically prepared. This primer is then annealed with the single-stranded DNA preparation, and subjected to DNA polymerizing enzymes such as E. coli polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as E. coli cells, and clones are selected that include recombinant vectors bearing the mutated sequence arrangement.

Alternatively, PCR™ directed mutagenesis of double-stranded DNA can be used. Such mutants may be readily prepared by, for example, directly synthesizing the Fc fragment by application of nucleic acid reproduction technology, such as the PCR™ technology of U.S. Patent 4,603,102 (incorporated herein by reference).

The preparation of sequence variants of the selected gene using site-directed mutagenesis is provided as a means of producing potentially useful species and is not meant to be limiting, as there are other ways in which sequence variants of genes may be obtained. For example, recombinant vectors encoding the desired gene may be treated with mutagenic agents, such as hydroxylamine, to obtain sequence variants.

### 8. Engineered IgG molecules with Extended In Vivo Half Lives

As noted above, the modified IgG molecules according to the present invention exhibit enhanced binding affinity for human FcRn and as a result, are likely to persist longer in serum than wild-type IgGs. The present invention provides a method for increasing the serum half life of an IgG molecule by providing a modified IgG molecule having an Fc domain or fragment thereof derived from IgG2, IgG3 or IgG4 comprising any of the combinations of amino acid residues described above.

Much interest has recently been directed towards modulating the properties (pharmacokinetics, distribution, etc.) of antibodies by site-directed mutagenesis by altering the strength of the FcRn-IgG interaction (Ghetie *et al,* 1997; Shields *et al,* 2001; Dall'Acqua *et al,* 2002; Hinton *et al,* 2004). The IgG residues involved in building the FcRn interaction site in both rodent and human IgGs have been identified (Table I and Section 3 above). These data do not rule out the involvement of additional residues of the Fc fragment in regulating IgG levels and FcRn-mediated transport, but it does provide a clear means by which the biological half-life, transport etc. of an antibody or antibody-based molecule or conjugate may be altered. It also provides a means by which the longevity of a particular antibody may be increased if desired, by re-inserting residues such as Ile253, His310, His435 and His436, should any such residues be found to be mutated in a particular antibody, e.g., IgG2b. Importantly, random mutagenesis of residues flanking these key amino acids, followed by selection, may yield an Fc fragment with increased serum half life (U.S. Patent 6,277,375; Ghetie *et al,* 1997; Hinton *et al,* 2004).

In accordance with the present invention, it has surprisingly been found that a particular combination of mutations gives rise to IgG molecules with unique properties. When

His433 is changed to Lys, and Asn434 is changed to Phe, but position 436 is retained in its wild-type form in the Fc domain of IgG2, IgG3 or IgG4, modified IgG molecules including such a domain or a fragment thereof show at least a 3-fold, typically a 4-6 fold higher binding affinity for human FcRn relative to wild-type IgG molecules belonging to the corresponding subclass from which the Fc domain derives. In addition, the pH dependence of IgG-FcRn binding is retained, and binding to CD16 is not reduced as compared with wild-type IgG molecules, indicating that effector functions of antibodies incorporating this combination of mutations will be retained. The claimed invention is broadly applicable to an almost unlimited number of therapeutic uses for the treatment of diseases or disorders, including but not limited to treatment of infectious diseases, autoimmunity, cancer and transplant rejection, as detailed in section 11 below.

### 9. Engineering IgG Molecules with FcRn Blocking Function

The present invention is also directed to methods for increasing IgG clearance rates in a subject and methods of blocking FcRn function in a subject comprising providing to said subject a modified IgG molecule according to the first aspect of the invention.

As described above, FcRn salvages IgG molecules from degradation and transports these molecules within and across cells. Blocking of FcRn binding to IgGs might therefore be desirable in situations where pathogenic antibodies (e.g., autoreactive) are present, or where an antibody is being used to clear a toxic drug or toxin from the body. Exposure to toxins such as snake venom can be treated with anti-venom antibodies. Similarly, overdoses with drugs such as digoxin are currently treated with anti-digoxin Fab fragments (DigiFab™). Following delivery of the antibody and complex formation with the toxin or drug, it might be desirable to induce rapid clearance of antibody-toxin complexes from the body. The current invention describes a modality of inducing rapid clearance of such complexes.

Modified IgG molecules according to the present invention may also be used as FcRn blocking agents to clear other types of undesirable antibodies from the circulation, for example antibodies that exhibit anti-drug properties. Administration of such blocking antibodies prior to or during administration of a therapeutic agent may be used to clear antibodies that neutralize the effects of such therapeutic agents from the circulation, so as to improve efficacy.

The modified IgG molecules according to the present invention bind FcRn with higher affinity than wild-type IgG molecules and can therefore be used as FcRn blocking antibodies. Such FcRn blocking antibodies have a large number of therapeutic uses for the treatment of disease or disorders, including but not limited to the treatment of autoimmunity or blockade of the transfer of pathogenic antibodies from mother to young.

### 10. Pharmaceutical Compositions

The modified IgG molecules according to the present invention may be formulated as pharmaceutical compositions suitable for administration to a subject, in particular a human subject. In addition to one or more IgG molecules as defined in accordance with the first aspect of the invention, the pharmaceutical composition includes a pharmaceutically acceptable buffer, carrier, diluent or excipient. Suitable agents would be known to those skilled in the art, and include excipients for modifying conditions such as pH, osmolarity, taste, viscosity, sterility, lipophilicity, solubility etc. Suitable agents with which the modified IgG molecules may be combined include surfactants or surface tension lowering agents, excipients that improve physiochemical stability or caking agents that promote freeze drying.

The pharmaceutical compositions described herein may be formulated in any suitable dosage form for any suitable route of delivery. Suitable dosage forms include solid dosage forms, for example, tablets, capsules, powders, dispersible granules, cachets and suppositories, including sustained release and delayed release formulations. Tablets, powders, cachets and capsules are all suitable dosage forms for oral administration.

Liquid dosage forms include solutions, suspensions and emulsions. Liquid form preparations may be administered by intravenous, intracerebral, intraperitoneal, parenteral or intramuscular injection or infusion. Sterile injectable formulations may comprise a sterile solution or suspension of the active agent in a non-toxic, pharmaceutically acceptable diluent or solvent. Suitable diluents and solvents include sterile water, Ringer's solution and isotonic sodium chloride solution, etc.

Liquid dosage forms also include solutions or sprays for intranasal administration.

Aerosol preparations suitable for inhalation may include solutions and solids in powder form, which may be combined with a pharmaceutically acceptable carrier, such as an inert compressed gas.

In certain embodiments, the pharmaceutical composition may be formulated for mucosal delivery, for example, formulated for intranasal administration. In certain embodiments, the pharmaceutical composition may be formulated for delivery via a tissue or epithelium that expresses FcRn. The epithelium may be selected from bronchial, intestinal or pulmonary epithelium. The binding of IgG molecules to FcRn expressed by particular tissues of the body may enhance the delivery and/or accumulation of the modified IgG molecules at certain sites, including muscle, skin and brain.

### 11. Therapeutic applications

The modified IgG molecules and pharmaceutical compositions as described herein may be used for a variety of different therapeutic applications.

### 11.1 Therapeutic antibodies

Therapeutic antibodies represent a major class of therapeutic agents used for the treatment of a variety of different diseases including cancer, Rheumatoid arthritis, Crohn's disease, asthma and a number of immunological disorders. Many therapeutic antibodies are already approved for use in the clinic, and many more are in the developmental pipeline. Therapeutic antibodies are typically monoclonal antibodies of murine or human origin. Murine therapeutic antibodies are frequently engineered so as to reduce their immunogenicity in humans, for example by antibody chimerization or humanization.

Although therapeutic antibodies may be produced and used as full-length immunoglobulin molecules, many variants have been developed. These variants include, but are not limited to, bi-specific antibodies, which are typically single antibody molecules capable of specific binding to two different target antigens, antibody-drug conjugates (ADCs) and intrabodies (therapeutic antibodies that act on intracellular targets).

The modified IgG molecules of the present invention may be incorporated into any of the therapeutic antibody forms as defined above, and that would be known to a person skilled in the art, provided the therapeutic antibody comprises an Fc domain or fragment thereof. Modified IgG molecules according to the present invention comprising an Fc domain or fragment thereof derived from IgG2, IgG3 or IgG4 wherein the Fc domain comprises the particular combination of amino acid residues Lys433, Phe434 and a wild-type amino acid residue at position 436 (mutant/mutant/wild-type), retain effector function and are therefore capable of triggering effector mechanisms such as ADCC.

Therapeutic antibodies are already used widely in the treatment of cancer, and many more anti-cancer antibodies are in pre-clinical and clinical development. The present invention provides methods of treating cancer using modified IgG molecules according to the present invention.

Cancer. The use of engineered antibodies to target cancers is an area of rapid expansion (Hudson and Souriau, 2003). Multiple antibodies for the treatment of various cancers have to date been approved by the FDA, e.g., Non-Hodgkin's lymphoma (anti-CD20; Rituxan™, Zevalin™), metastatic breast cancer (anti-Her-2; Herceptin™), acute myeloid leukemia (anti-CD33; Mylotarg™), chronic lymphocytic leukemia (anti-CD52; Campath™) and colon cancer (anti-VEGF: Avastin™).

Such antibodies would benefit from having an increased persistence *in vivo,* as this would result in the need for lower doses at less frequent intervals. Imaging of tumors with radiolabeled antibodies is also becoming increasingly common. The possibility of inducing the rapid clearance of an imaging agent from the body following imaging would be attractive as it would result in reduced radioactive burden.

Modified IgG molecules according to the present invention may also be used as a means to influence the biodistribution of therapeutic antibodies following administration to the human body. In particular, improved binding to FcRn may facilitate delivery to and/or accumulation of therapeutic antibodies in certain tissues, for example, skin, muscle and brain

### 11.2. Passive Immunotherapy

The present invention also includes a method of treating infection with a pathogenic agent using the modified IgG molecules described herein. Antibodies incorporating the combinations of mutated and wild-type residues described elsewhere herein retain effector function, and therefore effector mechanisms such as ADCC may be important in reducing or eliminating infection.

The protection of humans against infectious agents by passive immunotherapy is attractive, as it provides immediate protection. In contrast, active vaccination requires around 7-10 days for protective antibodies to be elicited. However, a major limitation of passive immunization is that protection is relatively short lived, due to clearance of protective antibodies from the circulation. Protective anti-pathogen antibodies that are longer lived in the circulation would therefore be attractive, and this invention relates directly to this application. Such antibodies would be useful in both therapy of infected individuals, as well as in prophylaxis in the case of likely exposure to an infectious agent. The administration of protective antibodies may also be important in the neutralization of biological agents that may be used as biological weapons, for example *Bacillus anthracis.*

In addition, infection of the newborn is of particular concern. The passive delivery of protective antibodies that occurs during the third trimester across the maternal-fetal barrier to the fetus could be utilized as a route to deliver protective antibodies, but the efficiency of transport is limited by competition by maternal IgGs (that may not be protective against the specific pathogen). This invention offers a way of increasing the competitive advantage of a therapeutic antibody over the endogenous, maternal antibodies in crossing the placenta.

Possible pathogens that might be targeted include, but are not limited to, Bacillus anthracis, West Nile virus, cytomegalovirus and respiratory syncytial virus.

### 11.3. Disease States with excessive and/or aberrant production of IgG

A number of disease states can result in the production of excessive or aberrant (i.e., pathologic) immunoglobulins. The present invention may, in one embodiment, be used to treat such diseases.

Therefore, in accordance with a further aspect of the invention, provided herein are methods of treating an autoimmune disease or disorder comprising administering to a subject in need thereof an effective amount of a modified IgG molecule as described herein.

The autoimmune diseases to be treated may be selected from those described below.

**Systemic lupus erythematosus (SLE).** SLE is an autoimmune rheumatic disease characterized by deposition in tissues of autoantibodies and immune complexes leading to tissue injury (Kotzin, 1996). In contrast to autoimmune diseases such as MS and type 1 diabetes mellitus, SLE potentially involves multiple organ systems directly, and its clinical manifestations are diverse and variable (Reviewed by Kotzin and O'Dell, 1995). For example, some patients may demonstrate primarily skin rash and joint pain, show spontaneous remissions, and require little medication. At the other end of the spectrum are patients who demonstrate severe and progressive kidney involvement that requires therapy with high doses of steroids and cytotoxic drugs such as cyclophosphamide (Kotzin, 1996). More recently, treatment with rituximab (anti-CD20; a B cell marker) has been attempted.

The serological hallmark of SLE, and the primary diagnostic test available, is elevated serum levels of IgG antibodies to constituents of the cell nucleus, such as double-stranded DNA (dsDNA), single-stranded DNA (ssDNA), and chromatin. Among these autoantibodies, IgG anti-dsDNA antibodies play a major role in the development of lupus glomerulonephritis (Tsao and Hahn, 1994; Ohnishi *et al,* 1994). Glomerulonephritis is a serious condition in which the capillary walls of the kidney's blood purifying glomeruli become thickened by accretions on the epithelial side of glomerular basement membranes. The disease is often chronic and progressive and may lead to eventual renal failure.

The mechanisms by which autoantibodies are induced in these autoimmune diseases remains unclear. As there has been no known cause of SLE, to which diagnosis and/or treatment could be directed, treatment has been directed to suppressing immune responses, for example with macrolide antibiotics, rather than to an underlying cause, (e.g., U.S. Patent 4,843,092).

**Rheumatoid Arthritis (RA).** The exact etiology of RA remains unknown, but the first signs of joint disease appear in the synovial lining layer, with proliferation of synovial fibroblasts and their attachment to the articular surface at the joint margin (Lipsky, 1998). Subsequently, macrophages, T cells and other inflammatory cells are recruited into the joint, where they produce a number of mediators, including the cytokines interleukin-1 (IL-I), which contributes to the chronic sequelae leading to bone and cartilage destruction, and tumour necrosis factor (TNF-α), which plays a role in inflammation (Dinarello, 1998; Arend and Dayer, 1995; van den Berg, 2001). The anti-TNFα antibody Remicade™ has been shown to have efficacy in the treatment of RA (Vilcek and Feldmann, 2004).

**Sjögren's Syndrome.** Sjögren's syndrome is a systemic autoimmune disease in which the body's immune system mistakenly attacks its own moisture producing glands. Sjögren's is one of the most prevalent autoimmune disorders, striking as many as 4,000,000 Americans, with 90% of patients being women. The average age of onset is late 40's although Sjögren's occurs in all age groups in both women and men. About 50% of the time Sjögren's syndrome occurs alone, and 50% of the time it occurs in the presence of another connective tissue disease. The four most common diagnoses that co-exist with Sjögren's syndrome are Rheumatoid Arthritis, Systemic Lupus, Systemic Sclerosis (scleroderma) and Polymyositis/Dermatomyositis. Sometimes researchers refer to these situations as "Secondary Sjögren's".

**Grave's Disease.** Marked by nervousness and overstimulation, Grave's disease is the result of an overactive thyroid gland (hyperthyroidism) due to production of autoreactive antibodies that recognize the thyroid stimulating hormone receptor. Thyroid hormones regulate metabolism and body temperature, and are essential for normal growth and fertility. But in excessive amounts, they can lead to the burn-out seen in this relatively common form of thyroid disease. It is unclear what triggers this problem, but the immune system is involved. In Grave's disease patients, they find antibodies specifically designed to stimulate the thyroid. In many cases, drugs that reduce thyroid output are sufficient to control the condition.

A short course of treatment with radioactive iodine, which dramatically reduces the activity of the thyroid, is another option for people past their childbearing years. In some cases, surgery to remove all or part of the thyroid (thyroidectomy) is needed. Surgery can also relieve some of the symptoms of Grave's disease. Bulging eyes, for example, can be corrected by creating enough extra space in the nearby sinus cavity to allow the eye to settle into a more normal position.

**Myasthenia Gravis.** The number of myasthenia gravis patients in the United States alone is estimated at .014% of the population, or approximately 36,000 cases; however, myasthenia gravis is likely under diagnosed. Myasthenia gravis is caused by autoreactive antibodies that recognize the acetylcholine (Ach) receptor. Previously, women appeared to be more often affected than men, with the most common age at onset being the second and third decades in women, and the seventh and eighth decades in men. As the population ages, the average age at onset has increased correspondingly, and now males are more often affected than females, and the onset of symptoms is usually after age 50.

In acquired myasthenia gravis, post-synaptic muscle membranes are distorted and simplified, having lost their normal folded shape. The concentration of ACh receptors on the muscle end-plate membrane is reduced, and antibodies are attached to the membrane. ACh is released normally, but its effect on the post-synaptic membrane is reduced. The post- junctional membrane is less sensitive to applied ACh, and the probability that any nerve impulse will cause a muscle action potential is reduced.

Modified IgG molecules according to the present invention may also be used to prevent and/or treat graft rejection following tissue or organ transplant. Tissue and organ grafts, though powerful tools for treating disease and injury, provoke powerful immune responses that can result in rapid graft rejection in the absence of immunosuppressive therapy. Pioneering studies conducted in the '40s and '50s established that allograft rejection was due to immune responses, later linked to T- lymphocytes. Specific immunological effector mechanisms responsible for graft rejection include cytotoxic T-cells, delayed-type hypersensitivity and antibody-dependent effects. Several therapeutic antibodies that target the T cell marker CD25 have been developed for the treatment of transplant rejection (e.g., Zenapax™, Simulect™). In addition, antibodies of the IgG classes can be involved in transplant rejection (Jordan, S. C. et al., 2005, Pediatric Transplantation, 9, 408-415). Blockade of FcRn by administration of modified IgG molecules according to the present invention might therefore be used to lower the levels of such deleterious antibodies during transplantation.

### 12. Use of modified IgG molecules as delivery agents

The IgG molecules according to the present invention may be used as delivery modules, for example delivery modules attached or conjugated to a secondary agent to be delivered to a subject, preferably a human subject. In certain embodiments, the IgG molecules for conjugation are Fc domains or fragments thereof. Conjugation of the IgG molecule to the secondary agent may be direct or indirect, for example mediated by any suitable linker moiety known to a person skilled in the art.

In certain embodiments, the secondary agent is a therapeutic or diagnostic agent to be administered to a subject. The therapeutic agent may be selected from any of the following:- an anti-bacterial anti-viral or anti-toxin agent. The diagnostic agent may be selected from a fluorescent label, a chemiluminescent label, a radiolabel or a chromophore.

### 13. Chimeric proteins

The present invention also relates to chimeric polypeptides or proteins comprising an IgG molecule according to the first aspect of the invention. As used herein the term "chimeric" polypeptide or protein refers to a polypeptide comprising a first amino acid sequence linked to a second amino acid sequence with which it is not naturally linked in nature. The amino acid sequences may normally exist in separate proteins that are brought together in the fusion polypeptide or they may normally exist in the same protein but are placed in a new arrangement in the fusion polypeptide. A chimeric polypeptide may be created, for example, by chemical synthesis, or by creating and translating a polynucleotide in which the peptide regions are encoded in the desired relationship.

Chimeric polypeptides can be prepared using any art recognised means. In certain embodiments, the chimeric polypeptides are prepared using recombinant DNA techniques. In other embodiments, the chimeric polypeptides are prepared by chemical synthesis.

Modified IgG molecules according to the present invention may be incorporated into chimeric polypeptides or fusion proteins in order to stabilize the polypeptide or sequence of amino acid residues to which the IgG is attached. The modified IgG molecule incorporated into the chimeric polypeptide may take any of the forms described elsewhere herein including a full-length heavy chain polypeptide or a fragment thereof, particularly wherein the fragment is an Fc domain of a heavy chain polypeptide. In certain embodiments, the IgG molecules of the present invention may be incorporated into fusion proteins so as to produce biologically stable therapeutic agents e.g. agents having an extended half-life. The IgG molecules of the present invention may also be used to stabilize polypeptides to which they are attached for the purposes of improving the efficiency of expression, for example in mammalian expression systems. The modified IgG molecules of the present invention are therefore proposed to be useful modules for both the stabilization and delivery of recombinant molecules, including chimeric proteins of therapeutic use.

In certain embodiments, the modified IgG molecules described herein may be incorporated into fusion proteins, particularly Fc fusion proteins called immunoadhesins. Immunoadhesins are chimeric, antibody-like molecules that combine the functional domain of a binding protein, for example a receptor, ligand, or cell-adhesion molecule with immunoglobulin Fc domains. Immunoadhesins incorporating the IgG molecules of the present invention may exhibit enhanced serum persistence and be beneficial in a number of therapeutic applications, as an alternative or in addition to therapeutic antibodies.

### 14. Transfer of IgG Across Membranes (Transcytosis)

As part of the present invention, it is contemplated that provision of antibodies in passive immunity will be achieved. In particular, improved transfer across cells and serum persistence of engineered antibodies is contemplated. In another embodiment, blockade of transport of IgGs within (recycling) or across (transcytosis) cells is envisaged by using IgGs that are designed to block FcRn function as described in section 8 above. A discussion of various transcellular transfer embodiments is provided below.

### 14.1. Intestinal Transfer in Newborns

An Fc receptor, now know to be FcRn, was originally implicated in transfer of IgG from the colostrum or milk into the bloodstream of newborn rats and mice (Brambell, 1966; Rodewald, 1976; Kim *et al.,* 1994). Consistent with the concept that FcRn is the transport receptor, maternal IgGs are not delivered to offspring in mice that do not express functional FcRn (Israel *et al.,* 1995). Thus, the available data indicate that IgG transcytosis in rats, mice and humans are carried out by similar receptors and as a consequence share a common mechanism. The proposed mechanism of trans-intestinal transport is similar to that described above (Section 2), with the exception that the intestinal lumen is at a pH (6.0-6.5) permissive for FcRn-IgG interactions. FcRn on the lumenal side of intestinal epithelial cells binds IgG and the IgG-FcRn complexes are transported across the cell to the basolateral surface where exocytosis occurs into the bloodstream of the newborn rodent. Association of IgG with FcRn as it traffics through the cell is postulated to protect the IgG molecule from lysosomal degradation. The pH of the plasma (pH7.3) results in release of the bound IgG into the circulation, consistent with the pH dependence of the FcRn-IgG interaction (Raghavan *et al,* 1995; Popov *et al,* 1996).

### 14.2. Transfer Across Murine Yolk Sac (Maternal-Fetal Transfer)

Murine FcRn is expressed at high levels in both neonatal intestine and yolk sac (Ahouse *et al.,* 1993), and an FcR that is structurally similar to FcRn has also been isolated from rat yolk sac (Roberts *et al.,* 1993). These data strongly suggested that maternal-fetal and intestinal transport are carried out by FcRn, although the cellular location for IgG binding to FcRn appears to differ in the two processes (Roberts *et al,* 1993). Consistent with the involvement of FcRn, maternal IgGs are not transported to the developing fetus in mice that lack functional FcRn (Israel *et al,* 1995). In rats, yolk sac FcRn is located in vesicles in the apical and basolateral cytoplasm, and not on the lumenal surface of the yolk sac endodermal cells (Roberts *et al,* 1993). The difference in location is believed to be necessary because the pH of the lumen surrounding the yolk sac is slightly basic (Roberts *et al,* 1993), and the affinity of binding of FcRn to IgG is low at this pH (Raghavan *et al,* 1995; Popov *et al,* 1996); thus, it has been suggested that maternal IgG is taken up by the yolk sac cells in a nonspecific endocytic step and then binds to FcRn in a slightly acidic endosomal compartment. Delivery of IgG into the fetal circulation is then proposed to occur in a similar way to that of intestinal transcytosis (Roberts *et al,* 1993).

More recent studies demonstrate that for transport of an IgG across the placenta, binding to human FcRn is essential (Firan *et al,* 2001), implicating FcRn in maternal-fetal transfer in humans. It is therefore expected that maternal transfer of passive immunity to infants will be improved if the affinity of the FcRn-IgG (or Fc) interaction is increased and serum persistence is lengthened. For enhanced serum persistence and maternal-fetal transfer of a therapeutic IgG, it is preferable to endow that IgG with a higher affinity for binding to the Fc receptors such as FcRn that are involved in the processes. As a result, the higher affinity IgGs should be able to out-compete the high concentrations of endogeneous IgGs (about 5 mg/ml in mice and 10 mg/ml in humans). Analogously, blockade of FcRn function using engineered IgGs (or fragments thereof) might be useful in preventing the transfer of deleterious maternal antibodies to the fetus.

### 14.3. FcRn-Mediated Transport Across Epithelial Barriers

FcRn was originally identified as the receptor that transports IgGs across the epithelial cells of neonatal intestine, where FcRn is highly expressed. However, more recent studies have shown that FcRn is functionally active in adult epithelial cells of diverse origin such as intestinal, bronchial and kidney epithelium (Spiekerman *et al,* 2002; Dickinson *et al,* 1999; Kobayashi *et al,* 2002; Yoshida *et al,* 2004). Significantly, FcRn can transport Fc-fusion proteins across bronchial epithelium (Spiekermann *et al,* 2002; Bitonti *et al,* 2004), and also plays a role in the delivery of antibody-antigen complexes across the gut (Yoshida *et al,* 2004). The latter process may play an important role in initiating immune responses against mucosal antigens. FcRn is therefore involved in transporting IgGs and their bound antigen (Yoshida *et al,* 2004) to diverse body sites throughout adult life.

The current invention has relevance to the transporting function of FcRn. For example, IgG molecules with improved binding properties for FcRn might be transported more efficiently across epithelia such as the adult intestinal epithelium or pulmonary epithelium, providing a route of delivery for therapeutic IgGs.

Alternatively, IgGs that block FcRn function might be useful for the blockade of, for example, bronchial FcRn that might relate to the blockade of allergic responses. Such IgGs may be administered via the bronchial epithelium.

The invention will be further understood with reference to the following non-limiting examples.

### EXAMPLES

### Example 1 Production of mutant IgG molecules with H433K/N434F mutation

The double mutation H433K/N434F (referred to herein as the "HN" mutation) was introduced into the Fc domain of different IgG subclasses (IgG2, IgG3 and IgG4) fused to the VH and VL of an anti-cMet antibody (mAb MAB1).

### 1.1 Cloning of IgG2, IgG3 and IgG4 in mammalian expression vector

The constant domains (CH1+Fc) of IgG2, IgG3 and IgG4 were cloned into a mammalian expression vector, starting from hIgG2 whose cDNA was ordered at geneart© (GenBank: BC062335), pFUSE-CHIg-hG3 (Invivogen, cat# pfuse-hchg301) and pFUSE-CHIg-hG4 (Invivogen, cat# pfuse-hchg4) respectively, using PCR and specific primers.

### 1.2 Introduction of the HN mutation by site-directed mutagenesis

After cloning the CH1+ Fc into the mammalian expression vector, the HN mutation H433K/N434F (as described in Vaccaro C *et al,* PNAS, 103, 2006) was introduced in the CH3 of IgG2, IgG3 and IgG4 by conventional site directed mutagenesis.

The primers used for IgG2 and IgG4 were:
5'gtgatgcatgaggctctgaagttccactacacacagaagagcctctccct 3'(sense primer) (SEQ ID NO: 1)
   and
5'agggagaggctcttctgtgtgtagtggaacttcagagcctcatgcatcac 3' (antisense primer) (SEQ ID NO: 2)

To introduce the HN mutation in IgG3, the primers were:
5'gtgatgcatgaggctctgaagttccgcttcacgcagaagagcctctccct3' (sense primer) (SEQ ID NO: 3)
   and
5'agggagaggctcttctgcgtgaagcggaacttcagagcctcatgcatcac3' (antisense primer) (SEQ ID NO: 4)

In the CH3 part of IgG3, an additional mutation (R435H) next to the HN mutation was introduced by site-directed mutagenesis using the primers:
5'gtgatgcatgaggctctgaagttccacttcacgcagaagagcctctccct3' (SEQ ID NO: 5)
and 5'agggagaggctcttctgcgtgaagtggaacttcagagcctcatgcatcac3' (SEQ ID NO: 6)

This mutation in combination with the HN mutation is referred to herein as "HN2" (H433K/N434F/R435H). Position 435 of IgG3 has previously been found to influence binding to FcRn (Stapleton *et al,* Nature Communications 2:599, December 2011).

An overview of the amino acid sequences of the Fc domains of the different IgG variants, in particular the regions flanking amino acid residues 433 and 434, is shown in Figure 3.

### 1.3 Ligation of the MAB1 variable domain into the different IgG variants (wild-type

### and mutants)

The variable domain of MAB1 (cMet specific mAb) was ligated into the constant domain of the different IgG variants (wild-type and HN/HN2 mutants) by PCR and conventional restriction sites. The final construct contains the signal peptide of the IgGkappa (MGWSCIILFLVATATGVHS) (SEQ ID NO: 24), the VH of MAB1 (anti-cMet) and the constant domains of the various IgG subclasses (CH1+hinge+Fc).

### 1.4 Transient production of modified IgGs in HEK293 cells

A DNA midiprep (Qiagen) was prepared and the complete heavy chain constructs (IgG2, IgG3 and IgG4) with and without the introduced mutations (HN or HN2) were used in combination to the MAB1 light chain transient transfection of HEK293 cells and transiently express the various mAb MAB1. All the antibodies were expressed in 100mL production scale.

The IgG2 and IgG4 mAbs purified with protein A and the IgG3 mAbs were purified with protein G. All mAbs were concentrated and a buffer exchange was done to PBS.

The yields after protein A or protein G purification are indicated in Table 2. The yields of the IgG3 mAbs were lower compared to the others but still normal expression levels were obtained. The presence of the HN mutation did not affect the production yield.

**Table 2: Yields of mAb MAB1 in the different IgG subclasses with and without the HN or HN2 mutations after purification.**

| | **mAb** | **Yield (µg/ml HEK cell)** |
|---|---|---|
| 1 | IgG2-MAB1 | 18.5 |
| 2 | IgG2-MAB1-HN | 17.9 |
| 3 | IgG3-MAB1 | 8.6 |
| 4 | IgG3-MAB1-HN | 7.3 |
| 5 | IgG3-MAB1-HN2 | 11.5 |
| 6 | IgG4-MAB1 | 25.9 |
| 7 | IgG4-MAB1-HN | 27.1 |

### 1.5 SDS PAGE analysis of the produced mAbs

All mAbs were analysed by SDS-PAGE. 2µg of each mAb was loaded on a precast gel (BioRad Ready Gel 4-20% Tris-HCl; cat# 161-1105) under non-reduced and reduced conditions (Figure 4, left and right panel respectively). The numbers refer to the same number in Table 2.

The purity and concentration of the mAbs was good. An extra band for IgG4 under non-reduced conditions was visible. This is probably a single HC-LC arm due to the well known phenomenon of IgG4 arm exchange, because the two arms of the IgG4 are not as tightly bound as the other IgGs. Under reduced conditions, it is clear that the heavy chain of IgG3 (wt, or with HN or HN2) is around 65kDa instead of 50kDa, due to the longer hinge fragment.

### Example 2 Functional Characterization of mutant IgG2, 3 and 4 molecules

### 2.1 Human decoy Met binding ELISA

The same variable region was used for all the mAbs, called MAB1. The functionality of the different purified mAbs was tested in a validated binding ELISA. A maxisorb plate was coated with 250ng/well decoy Met overnight at 4°C. After blocking with 2% BSA fr.V for 2h at room temperature, a dilution series of the mAbs (in PBS+0,1% BSA fr.V) was added and incubated for 1h at room temperatre on an orbital shaker. After washing, HRP-conjugated goat anti-human IgG Fc specific antibody (Jackson, cat# 109-035-008; diluted 1/10.000 in PBS+0,1% BSA fr.V) was added and incubated for 1h at room temperature. ABTS was added, incubated for 10min and the reaction was stopped with 1 N H₂SO₄. The optical density at 405nm was read and the values were represented in a graph against the concentration of the mAbs. Between every step, the plate was washed 5 times with PBS-Tween. Figure 5 shows the binding of the different mAbs to decoy Met and the obtained EC₅₀ values (µg/mL) are shown in Table 3.

**Table 3: EC₅₀ values (µg/mL) for the different mAbs**

| **mAb** | **EC₅₀ (µg/mL)** |
|---|---|
| IgG1-MAB1-P-HN | 0,018 |
| IgG2-MAB1 | 0,026 |
| IgG2-MAB1-HN | 0,028 |
| IgG3-MAB1 | 0,016 |
| IgG3-MAB1-HN | 0,016 |
| IgG3-MAB1-HN2 | 0,017 |
| IgG4-MAB1 | 0,031 |
| IgG4-MAB1-HN | 0,036 |

The HN mutation does not alter the binding activity of the antibody to the antigen (cMet). For IgG2 (+/-HN), IgG3 (+/-HN)2) and IgG4 (+/-HN), an EC₅₀ value of 0.026-0.028µg/mL, 0.016-0.017µg/mL and 0.031-0.036µg/mL was obtained respectively. The EC₅₀ value for the control afucosylated (-P) IgG1 molecule with the -HN mutation (MAB1-P-HN) is 0.018µg/mL and is in line with previous experiments (0.020-0.034µg/mL). The obtained EC₅₀ values indicate that the different subclasses are very similar to each other and do have similar potencies. The IgG2 and IgG4 antibodies appear to have lost a little bit of potency but this is still between the 2-fold error range.

### 2.2 Human CD16 binding ELISA

A binding ELISA experiment was performed to test if the binding to CD16 of the IgG subclasses was affected by the introduction of the HN or HN2 mutation. A maxisorb plate was coated with 100ng/well neutravidin overnight at 4°C. After blocking with 1% casein in PBS, 25ng/well biotinylated CD16 (Sino Biological Inc, cat#10389-H27H1-B; diluted in PBS+0.1% casein) was added and incubated for 1h at room temperature on an orbital shaker. A dilution series of the mAbs (in PBS+0,1% casein) was added and incubated for 1h before washing and addition of a HRP-conjugated goat anti-human Fab specific antibody (Sigma Aldrich, cat#A0293; diluted 1/50,000 in PBS+0,1% casein). After 1h incubation, s(HS)TMB was added, incubated for 10min and the reaction was stopped with 1 N H₂SO₄. The optical density at 450nm was read and the values were represented in a graph against the concentration of the mAbs. Between every step, the plate was washed 5 times with PBS-Tween. IgG1-MAB1-HN-P, an afucosylated antibody with the HN mutation, and Synagis, a normal IgG1 were used as positive control.

Figure 6 shows the results of the human CD16 ELISA for the different MAB1 mAbs. The obtained EC₅₀ values (µg/mL) are shown in Table 4.

**Table 4: EC₅₀ values (µg/mL) for the different mAbs**

| **mAb** | **EC₅₀ (µg/mL)** |
|---|---|
| IgG1 (Synagis) | 6.15 |
| IgG1-MAB1-HN-P (afucosylated) | 0.11 |
| IgG2-MAB1 | N.A. |
| IgG2-MAB1-HN | N.A. |
| IgG3-MAB1 | 3.58 |
| IgG3-MAB1-HN | 1.56 |
| IgG3-MAB1-HN2 | 1.26 |
| IgG4-MAB1 | N.A. |
| IgG4-MAB1-HN | N.A. |

As expected, no binding to CD16 was observed for IgG2 (with or without HN) and IgG4 (with and without HN).

The measured EC₅₀ values for IgG3 without or with the HN (or HN2) mutation vary between 1.26 and 3.58 µg/mL, respectively. The better affinity of the IgG3 to CD16 compared to a normal IgG1 (synagis, 6.15 µg/mL) is also expected since IgG3 is known to trigger more ADCC than IgG1. Interestingly, the addition of the HN or HN2 mutation seems to increase the binding to CD16 around 2.5-fold. However, the affinity measured in this experiment is in line with previous data on the normal IgG1-MAB1 (1.44-3.8µg/mL), which could indicate just internal variation of the assay.

The afucosylated IgG1-MAB1-HN-P with the-HN mutation was taken as a control and gives an EC₅₀ of 0.11 µg/mL which is in line with previous experiments. It was already reported that the afucosylated character of the antibody increases the affinity to human CD16.

### Example 3 FcRn binding in Biacore

### 3.1 FcRn binding using surface Plasmon resonance at pH 6.0

The HN mutations (H433K/N434F) have been reported to have increased affinity to human FcRn at pH6.0 but not at a pH7.2 and thereby increased half-life (Vaccaro C *et al,* PNAS, 103, 2006). It was described that the affinity to human FcRn for the HN mutant, measured with Surface Plasmon Resonance at pH6.0, was increased 15-fold compared to wild type IgG1 (34nM versus 528nM, Figure 7).

When the HN mutation was added to anti-IL6 or anti-cMet antibodies, it was subsequently found that the increase in affinity was more in the range of 5-6 fold. This difference is due to the differing methods used to measure the affinity of antibody binding. In the current experiments, a steady-state affinity fitting was used, which is different to the method used by Vaccaro *et al,* in which the authors used custom written software, which is not publicly available.

To investigate how the HN (or HN2) mutation in different subclasses of the mAb MAB1 influence the affinity for FcRn, the affinity was measured using Surface plasmon resonance (SPR) using the standard method used by Vaccaro *et al.* 750RU of each mAbs was immobilized on CM5 chips in NaAc buffer pH4.5 as shown in Table 5. When possible, an internal control, the afucosylated IgG1-HN antibody (IgG1-HN-P) was used.

**Table 5: Antibody MAB1 coated onto the different CM5-Chips**

| | Channel 1 (Fc1) | Channel 2 (Fc2) | Channel 3 (Fc3) | Channel 4 (Fc4) |
|---|---|---|---|---|
| IgG2 chip | - | IgG1-HN-P | IgG2 | IgG2-HN |
| IgG3 chip | - | IgG3 | IgG3-HN | IgG2-HN2 |
| IgG4 chip | - | IgG1-HN-P | IgG4 | IgG4-HN |

In two independent experiments, FcRn (obtained from S. Ward and confirmed functional) was added in 2-fold dilutions, starting at 4µM, in pH6.0 with a flow rate of 10µl/min. FcRn bound the different MAB1 variants (with and without HN) in a dose dependent fashion at pH6. The affinities (KD) were calculated after fitting the curves (steady-state fitting method) and showed an increased affinity (4.6 to 6.2-fold for the first experiment and 3.0 to 4.8-fold in the second experiments) for MAB1 (-P)-HN compared to MAB1 (Table 6).

It should be noted that the coated CM5 chips were stored at 4°C for a month between the first and second measurement which could explain the lower FcRn binding (RUs) obtained in the second experiment, resulting in lower affinities and consequently a lower fold improved affinity of the HN mutated mAbs vs. the wild type mAbs.

Although the affinity of FcRn for IgG1 was measured in parallel, the exact same conditions used in the first experiment (and notably the fact that the affinities were measured immediately after coating), allows the comparison of the affinities.

It is clear that all MAB1 mAbs of the different subclasses have a similar affinity to FcRn with 0.76, 0.87 and 0.95 µM for IgG2, IgG3 and IgG4, respectively. The similar affinity of the different subclasses to FcRn is also described in the literature and was comparable to what we observed (between 0.5 to 1µM) (West AP et al, Biochemistry, 39, 9698-9708, 2000 and Stapleton NM et al, Nature communication, Dec 2011).

**Table 6 Affinity of FcRn to MAB1 mAbs; determined in two different experiments**

| **mAb** | **KD pH6 (k_{off} / kₒₙ) µM (exp 1)** | **KD pH6 (k_{off} / kₒₙ) µM (exp 2)** | **Improved affinity vs. wild type mAb (exp1 and exp2)** |
|---|---|---|---|
| IgG1-MAB1-P-HN | 0.16 / 0.19 / 0.26 | 0.50/0.26 | - |
| | | | |
| IgG2-MAB1 | 0.76 | 1.53 | - |
| IgG2-HN-MAB1 | 0.14 | 0.51 | 5.4/3.00 |
| | | | |
| IgG3-MAB1 | 0.87 | 1.48 | - |
| IgG3-HN-MAB1 | 0.19 | 0.43 | 4.6/3.44 |
| IgG3-HN2-MAB1 | 0.14 | 0.31 | 6.2/4.77 |
| | | | |
| IgG4-MAB1 | 0.95 | 1.25 | - |
| IgG4-HN-MAB1 | 0.16 | 0.27 | 5.9/4.63 |
| | | | |
| *IgG1-MAB1* | *1.3* | | |
| *IgG1-HN-MAB1* | *0.25* | | *5.2* |

| | | | |
|---|---|---|---|
| Data mentioned in italics are from earlier experiments. | | | |

The introduction of the HN mutation improves the binding to FcRn 4 to 6-fold, to 140-160nM, at pH6.0 for all the IgG subclasses tested. It was increase 5.4 fold in IgG2 to 0.14 µM, 4.6 fold for IgG3 to 0.19 µM and 5.9 fold to 0.16 µM for IgG4.

The mutation of the Arginine to Histidine next to the HN in IgG3 (HN2 mutation) slightly improves the binding to FcRn as suggested in the article of Stapleton *et al.,* although it might not be very significant in this experiment.

### 3.2 FcRn binding using Surface Plasmon Resonance at pH 7.4

In another experiment using the same coated chip as described in Table 4, the binding to FcRn of the different MAB1 variants (with and without HN) was tested at pH7.4. Because of the very low affinity of IgG for FcRn at pH7.4, the variation in measurement is also higher. For example, almost no FcRn binding was observed to IgG1-MAB1-P-HN in the first Chip (10 RU), while some binding was observed on the other Chip (45RU, see two top sensograms of Figure 8).

Overall, only a slight increase in binding relative to that of the wild type MAB1 mAbs at pH7.4 was observed when very high concentration of FcRn (4uM) was used but it was still very minimal, and in the same range as observed for IgG1 (10-45 RU). The weak binding of FcRn (high concentration) at pH7 was also observed for IgG1 by Vaccaro C et al (PNAS, vol.103, no. 49, 2006), see Figure 7.

Figure 8 shows the binding of the different MAB1 mAbs to 4µM FcRn at both pH6.0 (upper line) and pH7.4 (lower line).

### Example 4 FcRn binding of modified IgG2 antibodies based on MAB2

A second set of modified IgG2 antibodies were generated, as described in Example 1 above for MAB1, using a second antibody, MAB2. MAB2 binds a different antigen to MAB1.

The modified IgG2 antibodies were produced so as to contain:
(i) a wild-type Fc region (MAB2-WT);
(ii) an Fc having the HN mutation described in Example 1 (MAB2-HN); and
(iii) an Fc having the HN mutation in addition to: M252Y/S254T/T256E (MAB2-HN/MST).

The binding of FcRn to the antibodies MAB2, MAB2-HN and MAB2-HN/MST was measured using Surface Plasmon Resonance (SPR) using the standard method of Vaccaro *et al.* (2005), and as described in Example 3.

712, 935 and 630RU of MAB2-WT, MAB2-HN and MAB2-HN/MST respectively, were immobilized on a CM5 chip. Then FcRn was added at different concentrations and affinities of FcRn at pH6.0 and pH7.4 to the different IgG2-MAB2s were determined as explained in Example 3. The results are shown in Table 7

**Table 7 Affinity of FcRn to MAB2 mAbs**

| | **KD(µM) pH6.0** | **KD(µM) pH7.4** |
|---|---|---|
| IgG2-MAB2-WT | 0.75 | ND(*) |
| IgG2-MAB2-HN | 0.087 | 6.9-63 (**) |
| IgG2-MAB2-HN/MST | 0.026 | 0.57 |

| | | |
|---|---|---|
| (*) ND: not binding detected (**) Estimation of affinity because the affinity is too low to be measured accurately under the conditions of the SPR experiments. | | |

The affinities at pH6.0 were consistent between IgG2-MAB1 (+/-HN) and IgG2-MAB2 (+/-HN), which confirms that the mutations have the same effect on IgG2 molecules irrespective of the antigen binding characteristics of the antibody.

Furthermore, it also shows that the introduction of the MST-HN mutation in a human IgG2 antibody increases significantly the binding to FcRn at pH6.0 as well as pH7.4 with affinity consistent with that of an IgG1-MST-HN (7.4nM at pH6.0 and 224nM at pH 7.2, see Vaccaro *et al.,* PNAS 2006).

These data indicate that hIgG2-HN-MST antibodies can function as FcRn blockers or "ABDEGs" (antibodies that enhance IgG degradation) to the same extent as has been shown for modified IgG1 antibodies (Vaccaro *et al.,* 2005).

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all embodiments described herein are considered to be broadly applicable and combinable with any and all other consistent embodiments, as appropriate.

Various publications are cited herein, the disclosures of which are incorporated by reference in their entireties.

### References

U.S. Patent 4,843,092
U.S. Patent 6,277,375
Antohe et al, Human Immunol, 62:93-105, 2001.
Arend and Dayer, Arthritis Rheum., 38:151-160, 1995.
Bitonti et al, Proc. Natl. Acad. ScL, USA, 101 :9763-9768, 2004.
Burmeister et al, Nature, 336-343, 1994b.
Dall' Acqua et al, J. Immunol, 169:5171-5180, 2002.
Dickinson et al, J. CUn. Invest., 104, 903-911, 1999.
Dinarello, Int. Rev. Immunol, 16:457-499, 1998.
Edelman et al, Proc. Natl. Acad. ScL, USA, 63:78-85, 1969.
Firan et al, Int. Immunol, 13(8):993-1002, 2001.
Foote and Winter, J. Mol. Biol, 224:487-499, 1992.
Ghetie and Ward, Anmi. Ref. Immunol, 18:739, 2000.
Ghetie et al, Eur. J. Immunol, 26(3):690-696, 1996.
Ghetie et al, Immunol Today, 18(12):592-598, 1997.
Hinton et al, J. Biol Chem., 279:6213-6216, 2004.
Horton et al, Gene, 77:61-68, 1989.
Hudson and Souriau, Nat. Med., 9:129-134, 2003.
Israel et al, Immunol, 89:573-578, 1996.
Israel etal, J. Immunol, 154:6246-6251, 1995.
Junghans and Anderson, Proc. Natl. Acad. ScL USA, 93(11):5512-5516, 1996.
Kabat et al, In: Sequences of proteins of immunological interest, U.S. Department of Health and Human Services, 1991.
Kacskovics et al, J. Immunol, 164:1889-1897, 2000.
Kim et al, Eur. J. Immunol, 24:2429-2434, 1994a.
Kim et al, Eur. J. Immunol, 24:542-548, 1994b.
Kim et al, Scand. J. Immunol, 40:457-465, 1994c.
Kim et al, Eur. J. Immunol, 29:2819-2825, 1999.
Kobayashi et al, Am. J. Physiol. Renal Physiol, 282, F358-F365, 2002.
Kotzin and O'Dell, In: Samler's Immunologic Diseases, 5th Ed., Frank et al. (Eds.), Little Brown & Co., Boston, 667-697, 1995. Kotzin, Cell, 85:303-306, 1996.
Kristoffersen and Matre, Eur. J. Immunol, 26(7): 1668- 1671, 1996.
Leach et al, J. Immunol, 157:3317-3322, 1996.
Lipsky, In: Harrison's principles of internal medicine, Fauci et al.(Eds.), 14th Ed., NY, McGraw-Hill, 1880-1888, 1998.
Martin et al, Mol Cell, 7:867-877, 2001.
Martin et al., Molecular Cell, 7:867-877, 2001.
McCarthy et al, J. Cell ScL, 113:1277-1285, 2000.
Medesan et al, Eur. J. Immunol, 26:2533-2536, 1996.
Medesan etal, J. Immunol, 158(5):2211-2217, 1997.
Ober et al, Int. Immunol, 13:1551-1559, 2001.
Ober et al, J. Immunol, 172:2021-2029, 2004.
Ober et al, J. Immunol, .172(4) :2021-2029, 2004.
Ober et al, Proc. Natl Acad. ScL USA, 101 (30):11076-I 1081, 2004.
Ober et al, Proc. Natl. Acad. Sci, USA, 101:11076-11081, 2004.
Ohnishi et al, Nephrol Dial Transplant., 9:1747-1750, 1994.
Popov et al, Mol Immol, 33:521, 1996.
Raghavan et al, Biochemistry, 34:14649, 1995.
Roberts et al, J. Cell Biol, 111 :1867-1876, 1990.
Rodewald and Kraehenbuhl, J. Cell Biol, 99:S159-S164, 1984.
Shields et al, J. Biol. Chem., 276:6591-6604, 2001.
Simister et al, Eur. J. Immunol, 26(7):1527-1531, 1996.
Simister, Vaccine, 21 :3365-3369, 2003.
Spiekermann et al, J. Exp. Med., 196(3):303-310, 2002.
Stapleton NM et al, Nature communication, December 2011.
Story et al, J. Exp. Med, 180:2377-2381, 1994.
Tsao and Hahn, Int Rev Immunol, 11 (4):305-320, 1994.
Vaccaro et al, Nat. Biotech., 23, 1283-1288, 2005.
Vaccaro C et al, PNAS, 103, 2006.
van den Berg, Semin. Arthritis Rheum., 30(5S-2):7-16, 2001.
Vilcek and Feldmann, Trends in Pharmacological Sciences, 25:201-209, 2004.
Wallace and Rees, Biochem. J, 188:9-16, 1980.
West AP et al, Biochemistry, 39, 9698-9708, 2000
Yoshida et al, Immunity, 20:769-783, 2004.
Zhou etal, J. Mol Biol, 332:901-913, 2003.
Zhou etal, J. Mol. Biol, 345, 1071-1081, 2005.

The invention will be further understood with reference to the following numbered clauses.
1. An IgG molecule comprising an Fc domain or a fragment thereof derived from IgG2, IgG3 or IgG4, wherein the Fc domain comprises Lys433, Phe434 and a wild-type amino acid residue at position 436.
2. An IgG molecule according to clause 1 wherein the Fc domain or fragment thereof is derived from IgG2 or IgG4 and includes Tyr436.
3. An IgG molecule according to clause 1 wherein the Fc domain or fragment thereof is derived from IgG3 and includes Phe436.
4. An IgG molecule according to any of clauses 1-3 wherein the Fc domain further comprises His435.
5. An IgG molecule according to any of clauses 1-4 wherein the Fc domain or fragment thereof is derived from human IgG2, IgG3 or IgG4.
6. An IgG molecule according to any of clauses 1-5 wherein the Fc domain further comprises one or more of Tyr252, Thr254 and Glu256.
7. An IgG molecule according to any of clauses 1-5 wherein the Fc domain further comprises Tyr252.
8. An IgG molecule according to any of clauses 1-5 wherein the Fc domain further comprises Thr254.
9. An IgG molecule according to any of clauses 1-5 wherein the Fc domain further comprises Glu256.
10. An IgG molecule according to any of clauses 1-5 wherein the Fc domain further comprises Tyr252 and Thr254.
11. An IgG molecule according to any of clauses 1-5 wherein the Fc domain further comprises Tyr252 and Glu256.
12. An IgG molecule according to any of clauses 1-5 wherein the Fc domain further comprises Thr254 and Glu256.
13. An IgG molecule according to any of clauses 1-5 wherein the Fc domain further comprises Tyr252, Thr254 and Glu256.
14. An IgG molecule according to any of clauses 1-13 wherein the IgG molecule is selected from the group consisting of:- a bivalent immunoglobulin; a single chain immunoglobulin; a full-length IgG heavy chain polypeptide; and a fragment of an IgG heavy chain polypeptide.
15. An IgG molecule according to clause 14 wherein the IgG molecule is a fragment of an IgG heavy chain polypeptide and the fragment is a peptide of at least 40 amino acid residues in length.
16. An IgG molecule according to any of clauses 1-14 wherein the IgG is capable of binding to an antigen.
17. An IgG molecule according to clause 16 wherein the IgG molecule binds to a pathogen antigen.
18. An IgG molecule according to clause 17 wherein the pathogen is a virus, bacterium or fungus.
19. An IgG molecule according to clause 17 wherein the pathogen is a parasite.
20. An IgG molecule according to any of clauses 1-14 wherein the IgG molecule is capable of binding to a toxin.
21. An IgG molecule according to any of clauses 1-14 wherein the IgG molecule is capable of binding to an autoimmune antibody or an anti-transplant antibody.
22. A polynucleotide sequence encoding an IgG molecule as defined in any of clauses 1-21.
23. An expression vector including a polynucleotide sequence according to clause 22.
24. A host cell comprising an expression vector according to clause 23.
25. An IgG molecule according to any of clauses 1-21 wherein the IgG molecule is conjugated to a therapeutic or diagnostic agent.
26. An IgG molecule according to clause 25 wherein the IgG molecule is an Fc domain or fragment thereof.
27. An IgG molecule according to clause 25 or clause 26 wherein said therapeutic agent is an anti-bacterial, anti-viral or anti-toxin.
28. An IgG molecule according to clause 25 or clause 26 wherein said diagnostic agent is a fluorescent label, a chemiluminescent label, a radiolabel or a chromophore.
29. An IgG molecule according to any of clauses 1-21 for use as a medicament.
30. An IgG molecule for use according to clause 29 wherein the IgG molecule is formulated for delivery via an epithelium that expresses FcRn.
31. An IgG molecule for use according to clause 30 wherein the epithelium is selected from bronchial, intestinal or pulmonary epithelium.
32. A pharmaceutical composition comprising an IgG molecule as defined in any of clauses 1-21 and a pharmaceutically acceptable buffer, carrier, diluent or excipient.
33. A pharmaceutical composition according to clause 32 wherein the composition is formulated for mucosal delivery.
34. A pharmaceutical composition according to clause 32 wherein the composition is formulated for delivery via an epithelium that expresses FcRn.
35. A pharmaceutical composition according to clause 34 wherein the epithelium is selected from bronchial, intestinal or pulmonary epithelium.
36. A method of treating an autoimmune disease or disorder comprising administering to a subject in need thereof an effective amount of an IgG molecule according to any of clauses 1 to 21 or an effective amount of a pharmaceutical composition according to any of clauses 32 to 35.
37. A method according to clause 36 wherein the autoimmune disease/disorder is selected from the following:- Systemic Lupus Erythematosus, Rheumatoid Arthritis, Sjögren's Syndrome, Grave's disease, and Myasthenia Gravis.
38. A method of treating infection with a pathogenic agent comprising administering to a subject in need thereof an effective amount of an IgG molecule according to any of clauses 1 to 21 or an effective amount of a pharmaceutical composition according to any of clauses 32 to 35.
39. A method of treating cancer comprising administering to a subject in need thereof an effective amount of an IgG molecule according to any of clauses 1 to 21 or an effective amount of a pharmaceutical composition according to any of clauses 32 to 35.
40. A method for increasing the serum half life of an IgG molecule in a subject comprising providing said subject with an IgG molecule according to any of clauses 1-21 or a pharmaceutical composition according to any of clauses 32 to 35.
41. A method of increasing IgG clearance rates in a subject comprising providing to said subject an IgG molecule according to any of clauses 1-21 or a pharmaceutical composition according to any of clauses 32 to 35.
42. A method of blocking FcRn function in a subject comprising providing to said subject an IgG molecule according to any of clauses 1-21 or a pharmaceutical composition according to any of clauses 32 to 35.
43. A method of providing an IgG molecule to a fetus comprising the steps of: (i) providing an IgG molecule according to any of clauses 1-21 and (ii) administering the IgG molecule to a subject carrying a fetus, wherein said IgG molecule is transported across the placental membrane of the subject to the fetus.
44. A chimeric protein comprising an IgG molecule as defined in any of clauses 1-21.
45. A chimeric protein according to clause 44 wherein the IgG molecule is an Fc domain or fragment thereof.
46. A chimeric protein according to clause 44 wherein the protein is an immunoadhesin.

## Claims

1. An IgG molecule comprising an Fc domain or a fragment thereof derived from IgG2, IgG3 or IgG4, wherein the Fc domain comprises Lys433, Phe434 and a wild-type amino acid residue at position 436.

2. An IgG molecule according to claim 1 wherein the Fc domain or fragment thereof is derived from IgG2 or IgG4 and includes Tyr436 or wherein the Fc domain or fragment thereof is derived from IgG3 and includes Phe436.

3. An IgG molecule according to claim 1 or claim 2 wherein the Fc domain further comprises His435 and/or wherein the Fc domain or fragment thereof is derived from human IgG2, IgG3 or IgG4.

4. An IgG molecule according to any of claims 1-3 wherein the Fc domain further comprises one or more of Tyr252, Thr254 and Glu256, or wherein the Fc domain further comprises Tyr252 or Thr254 or Glu256, or wherein the Fc domain further comprises Tyr252 and Thr254 or Tyr252 and Glu256 or Thr254 and Glu256, or wherein the Fc domain further comprises Tyr252, Thr254 and Glu256.

5. An IgG molecule according to any of claims 1-4 wherein the IgG molecule is selected from the group consisting of:- a bivalent immunoglobulin; a single chain immunoglobulin; a full-length IgG heavy chain polypeptide; and a fragment of an IgG heavy chain polypeptide, optionally wherein the IgG molecule is a fragment of an IgG heavy chain polypeptide and the fragment is a peptide of at least 40 amino acid residues in length.

6. An IgG molecule according to any of claims 1-5 wherein the IgG is capable of binding to an antigen, optionally a pathogen antigen, optionally wherein the pathogen is a virus, bacterium or fungus or wherein the pathogen is a parasite.

7. An IgG molecule according to any of claims 1-6 wherein the IgG molecule is capable of binding to a toxin or wherein the IgG molecule is capable of binding to an autoimmune antibody or an anti-transplant antibody.

8. A polynucleotide sequence encoding an IgG molecule as defined in any of claims 1-7.

9. An expression vector including a polynucleotide sequence according to claim 8.

10. A host cell comprising an expression vector according to claim 9.

11. An IgG molecule according to any of claims 1-7 wherein the IgG molecule is conjugated to a therapeutic or diagnostic agent, optionally wherein the IgG molecule is an Fc domain or fragment thereof, and optionally wherein said therapeutic agent is an anti-bacterial, anti-viral or anti-toxin or optionally wherein said diagnostic agent is a fluorescent label, a chemiluminescent label, a radiolabel or a chromophore.

12. An IgG molecule according to any of claims 1-11 for use as a medicament, optionally wherein the IgG molecule is formulated for delivery via an epithelium that expresses FcRn, optionally wherein the epithelium is selected from bronchial, intestinal or pulmonary epithelium.

13. A pharmaceutical composition comprising an IgG molecule as defined in any of claims 1-7 and a pharmaceutically acceptable buffer, carrier, diluent or excipient, optionally wherein the composition is formulated for mucosal delivery or optionally wherein the composition is formulated for delivery via an epithelium that expresses FcRn, optionally wherein the epithelium is selected from bronchial, intestinal or pulmonary epithelium.

14. A chimeric protein comprising an IgG molecule as defined in any of claims 1-7, optionally wherein the IgG molecule is an Fc domain or fragment thereof, or optionally wherein the protein is an immunoadhesin.

15. A method for increasing the serum half life of an IgG molecule in a subject comprising providing said subject with an IgG molecule according to any of claims 1-7 or a pharmaceutical composition according to claim 13.

16. A method of increasing IgG clearance rates in a subject comprising providing to said subject an IgG molecule according to any of claims 1-7 or a pharmaceutical composition according to claim 13.

17. A method of blocking FcRn function in a subject comprising providing to said subject an IgG molecule according to any of claims 1-7 or a pharmaceutical composition according to claim 13.

18. A method of providing an IgG molecule to a fetus comprising the steps of: (i) providing an IgG molecule according to any of claims 1-7 and (ii) administering the IgG molecule to a subject carrying a fetus, wherein said IgG molecule is transported across the placental membrane of the subject to the fetus.

19. An IgG molecule according to any of claims 1-7 or a pharmaceutical composition according to claim 13 for use in the treatment of an autoimmune disease or disorder, optionally wherein the autoimmune disease/disorder is selected from the following:-Systemic Lupus Erythematosus, Rheumatoid Arthritis, Sjögren's Syndrome, Grave's disease, and Myasthenia Gravis, and wherein the treatment comprises administering to a subject in need thereof an effective amount of the IgG molecule or an effective amount of the pharmaceutical composition.

20. An IgG molecule according to any of claims 1-7 or a pharmaceutical composition according to claim 13 for use in the treatment of infection with a pathogenic agent wherein the treatment comprises administering to a subject in need thereof an effective amount of the IgG molecule or an effective amount of the pharmaceutical composition.

21. An IgG molecule according to any of claims 1-7 or a pharmaceutical composition according to claim 13 for use in the treatment of cancer wherein the treatment comprises administering to a subject in need thereof an effective amount of the IgG molecule or an effective amount of the pharmaceutical composition.
